(19) Europäisches Patentamt
European Patent Office
Office européen des brevets

(11) EP 4 135 623 B1

(12) # EUROPEAN PATENT SPECIFICATION

(45) Date of publication and mention of the grant of the patent:
**08.04.2026 Bulletin 2026/15**

(21) Application number: **21788921.1**

(22) Date of filing: **15.04.2021**

(51) International Patent Classification (IPC):
***A61L 27/16*** (2006.01) ***A61L 27/18*** (2006.01)
***A61L 27/50*** (2006.01) ***A61F 2/16*** (2006.01)
***A61F 2/00*** (2006.01) ***C08F 220/00*** (2006.01)
***C08L 83/04*** (2006.01) ***C09J 183/04*** (2006.01)

(52) Cooperative Patent Classification (CPC):
(C-Sets available)
**A61F 2/1654; A61L 27/16; A61L 27/18; A61L 27/50; C09J 183/04;** A61F 2002/1681; A61F 2002/16965; A61F 2240/002; A61F 2250/0004; A61L 2430/16; C08G 77/12; C08G 77/20 (Cont.)

(86) International application number:
**PCT/US2021/027546**

(87) International publication number:
**WO 2021/211888 (21.10.2021 Gazette 2021/42)**

(54) **COMPOSITE LIGHT ADJUSTABLE INTRAOCULAR LENS WITH ADHESION PROMOTER**

ZUSAMMENGESETZTE LICHTANPASSBARE INTRAOKULARLINSE MIT HAFTVERMITTLER

LENTILLE INTRAOCULAIRE RÉGLABLE À LA LUMIÈRE COMPOSITE DOTÉE D'UN PROMOTEUR D'ADHÉRENCE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.04.2020 US 202016849556**

(43) Date of publication of application:
**22.02.2023 Bulletin 2023/08**

(73) Proprietor: **RxSight, Inc.**
**Aliso Viejo, CA 92656 (US)**

(72) Inventors:
- **SHRESTHA, Ritu**
  **Huntington Beach, CA 92647 (US)**
- **GOLDSHLEGER, Ilya**
  **Ladera Ranch, CA 92694 (US)**
- **KONDIS, John**
  **Irvine, CA 92614 (US)**
- **KURTZ, Ronald, M.**
  **Irvine, CA 92603 (US)**

(74) Representative: **J A Kemp LLP**
**80 Turnmill Street**
**London EC1M 5QU (GB)**

(56) References cited:
**US-A- 5 395 872**
**US-A1- 2003 116 273**
**US-A1- 2007 129 802**
**US-A1- 2012 245 684**
**US-A1- 2018 338 827**
**US-B2- 8 969 429**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61L 27/16, C08L 33/08;**
**A61L 27/18, C08L 83/04;**
**C09J 183/04, C08L 83/00**

## Description

### CROSS-REFERENCE TO RELATED APPLICATION

This application is a continuation-in-part of co-pending patent application US 15/607,681, entitled: "Composite Light Adjustable Intraocular Lens", by I. Goldshleger, J. Kondis, R.M. Kurtz, and R. Shrestha.

### TECHNICAL FIELD

This invention relates to light adjustable intraocular lenses, and more specifically to composite intraocular lenses that can be adjusted by illumination.

### BACKGROUND

The techniques of cataract surgery have been experiencing continuous, impressive progress of late. Subsequent generations of phacoemulsification platforms and newly invented surgical lasers keep increasing the precision of the placement of intraocular lenses (IOLs) and keep reducing the unwanted medical outcomes. Also, present generations of IOLs, based on soft acrylate materials, deliver very good optical outcomes, and numerous additional medical benefits, including ease and control of the implantation process, and an advantageous haptic design.

Nevertheless, some types of challenges remain even with the latest generation of devices and IOLs. One of them is that, in spite of surgeons carrying out the most careful pre-surgical diagnostics to determine the optimal IOL to be implanted, in a notable percentage of cases the post-surgical medical outcomes are less than optimal. This can be caused by a variety of factors, including an uneven healing process of the incisions tilting or moving the implanted IOL, or an imperfect modeling of the eye, among others.

A noteworthy breakthrough has been achieved recently by the development of lenses that can be adjusted non-invasively after the cataract surgery. These lenses involve light sensitive materials that photopolymerize upon activation by an irradiation. Irradiation with a carefully designed radial profile initiates the photopolymerization with a corresponding radial profile, which, in turn, leads to the IOL changing its physical shape and therefore, its optical power. These light adjustable lenses hold great promise to adjust and eliminate the residual post-surgical misalignments and to fine tune "the last diopter" of the IOLs post-surgically and non-invasively.

US 2018/338827 A1 discloses a composite light adjustable intraocular lens that includes an intraocular lens, a light adjustable lens, attached to the intraocular lens; and haptics. The light adjustable lens can be attached to the intraocular lens by a chemical reaction, a thermal treatment, an illumination treatment, a polymerization process, a molding step, a curing step, a lathing step, a cryo-lathing step, a mechanical process, an application of an adhesive, or by any combination of these methods. However, the present generation of these light adjustable lenses can be further improved still. Areas of possible improvements include optimized material properties that could ease the challenges of the implantation, as well as better haptic designs.

Therefore, there is an unmet medical need for intraocular lenses that deliver the advantages of both today's regular acrylate IOLs, and that of the light adjustable IOLs, while minimizing the less desirable aspects of their performance.

### SUMMARY

In this patent document, the above-described needs are addressed by embodiments of a composite light adjustable intraocular lens that include an acrylic intraocular insert; a silicone-based light adjustable lens, attached to the acrylic intraocular insert; and haptics, as described in claim 1. In some cases, a composite light adjustable intraocular lens can include an intraocular lens, and haptics, attached to the IOL with light-adjustable hinges. Also described but not claimed is a method of adjusting an implanted composite light adjustable intraocular lens which can include planning a targeted optical outcome of an implantation of the composite light adjustable intraocular lens into an eye; implanting the composite light adjustable intraocular lens into the eye; performing a diagnostic measurement to evaluate an implanted optical outcome of the implantation; determining a correction based on a comparison of the planned optical outcome and the implanted optical outcome; and applying a stimulus to adjust an optical characteristic of the composite light adjustable intraocular lens to induce the determined correction.

### BRIEF DESCRIPTION OF THE DRAWINGS

**FIG. 1** illustrates a top view of a composite light adjustable IOL.
**FIGS. 2A-C** illustrate side views of embodiments of a composite light adjustable IOL, or CLA IOL.
**FIG. 3** illustrates a side view of another embodiment of a composite light adjustable IOL.
**FIG. 4** illustrates steps of a light adjustment procedure.
**FIG. 5.** illustrates an embodiment of a composite light adjustable IOL with a UV absorber layer.
**FIG. 6.** illustrates a CLA IOL with an attachment structure.
**FIGS. 7A-C** illustrate a formation of a counter-rotating toric pattern in an implanted rotated toric CLA IOL.
**FIGS. 8A-C** illustrate the formation of an analogous counter-rotating cylinder using a vector formulation.
**FIG. 9** illustrates a method of adjusting a composite light adjustable IOL.
**FIGS. 10A-B** illustrate chromatic aberration-reducing CLA IOLs.
**FIG. 11** illustrates the chromatic shift of a CLA IOL, in comparison to a regular IOL.
**FIGS. 12A-B** illustrate PCO-suppressing aspects of an achromatic embodiment of the composite light adjustable IOL.
**FIG. 13** illustrates embodiment of the composite light adjustable IOL with an adhesion promoter.
**FIGS. 14A-C** illustrate cross-sectional views of embodiments of the composite light adjustable IOL.
**FIGS. 15A-B** illustrate embodiments of the composite light adjustable IOL with different incorporations of the adhesion promoter.
**FIGS. 16A-B** illustrate embodiments of the composite light adjustable IOL with different incorporations of the adhesion promoter.
**FIGS. 17A-C** illustrate embodiments of the composite light adjustable IOL with different incorporations of the adhesion promoter.
**FIGS. 18A-B** illustrate embodiments of the composite light adjustable IOL with differently positioned UV absorbing layers.
**FIGS. 19A-B** illustrate embodiments of the composite light adjustable IOL with the acrylic intraocular insert having a diffractive structure.

## DETAILED DESCRIPTION

Existing light adjustable intraocular lenses are often made of silicone-based polymers, such as poly-siloxanes and corresponding copolymers. Existing non-light-adjustable intraocular lenses are often made of various acrylates. The limitations (L) and the benefits (B) of these two classes of IOLs include the followings.

(L1) The elastic constants of silicone-based IOLs are often stronger, or stiffer, than that of some other IOL's, and therefore these silicone-based IOLs are often "springy" in comparison. One consequence of this springiness is that, during the IOL implantation process, the folded silicone-based IOLs unfold quite fast as they are pushed out from the surgical inserter hand piece into the eye. This quick unfolding of the silicone-based IOLs can make the control of the insertion and the proper alignment of the silicone-based IOLs somewhat challenging for a surgeon during surgery.

(B1) In contrast, acrylate-based IOLs have softer elastic constants, and thus unfold slower during the insertion. This aspect allows the surgeon to exercise more control over the insertion of acrylate IOLs.

(L2) The design of silicone-based IOLs is often three-piece: the two haptics are often separately fabricated and subsequently inserted into the central lens body. This design feature increases manufacturing costs, may lead to a higher rate of haptics misalignment during manufacture, and to separation of the haptics from the IOL lens body during the insertion.

(B2) In contrast, some acrylate-based IOLs manage these challenges by having a one-piece design, where integrated haptics are formed from the same lens material and with the same molding step as the central lens body of the IOL. Such one-piece designs have lower manufacturing costs, deliver good haptics alignment with the lens body, and reduce the risk of haptic separation from the lens body during insertion.

At the same time, the presently known acrylate-based IOLs are not light adjustable. These non-light adjustable, often acrylate-based IOLs have drawbacks on their own. These include the followings.

(L3) When surgeons plan a cataract surgery, first they perform careful and extensive diagnostics of the cataractous eye. Based on this diagnostics, the surgeons determine the optimal placement, alignment and optical power of the IOL. However, as discussed previously, the IOLs often end up away from their planned optimal placement, possibly tilted or misaligned relative to the plan. This can happen for a variety of causes, such as uneven development of ocular tissue after the surgery.

(B3) Light adjustable IOLs offer a profound solution for this misplacement and misalignment problem. Once the IOL is implanted and settled in the capsular bag of the eye after surgery, a post-surgical diagnostics can be carried out to determine the unintended shifts in alignment and placement of the implanted IOL. The results of this post-surgical diagnostics can be used to determine what corrections of the IOL can compensate the misplacement and the misalignment of the implanted IOL. This post-surgical determination can be used to perform a light adjustment procedure to bring about the determined IOL corrections in the implanted light adjustable IOL.

(L4) The above misalignment problem is particularly acute for toric IOLs, where the implantation targets the elimination of a cylinder in the eye. For toric IOLs, an unintended rotation of the toric IOL axis by only 10 degrees after implantation can cause about 30% loss of efficiency. E.g. a nominal 3D cylinder of a toric IOL can be reduced to an effective 2D cylinder if the cylinder axis ends up rotated by only 10 degrees during or after implantation.

(B4) Light adjustable IOLs can be implanted without any preformed toric cylinder. After the implantation, when the IOL settled and stopped its unintended rotation, the surgeon can apply an illumination to form a cylinder in the settled IOL, with its axis oriented exactly in the planned or targeted direction. Thus, light adjustable IOLs are capable of avoiding the possible loss of efficiency induced by unintended misalignments of the cylinder axis of toric IOLs.

This document describes intraocular lenses that combine the benefits (B1)-(B4) of the above two classes of IOLs, and therefore have the potential to overcome and to avoid the limitations (L1)-(L4) each class of IOLs has on their own. Additional benefits of various embodiments will be also articulated below.

**FIG. 1** illustrates a top view of a composite light adjustable intraocular lens 100 that includes an intraocular lens (IOL) 110, a light adjustable lens (LAL) 120, attached to the intraocular lens 110; and haptics 114-1 and 114-2, cumulatively also referred to as haptics 114. The haptics 114 can include various number of haptic arms. Embodiments with one, two, three and more haptic arms all have their advantages. For compactness and specificity, the rest of the description is directed to composite light adjustable intraocular lenses 100 with two haptic arms 114-1 and 114-2, but embodiments with other number of haptic arms are understood to be within the scope of the overall description.

**FIGS. 2A-C** and **FIG. 3** illustrate side views of embodiments of the composite light adjustable intraocular lens 100, or CLA IOL 100. **FIGS. 2A-C** illustrate a CLA IOL 100 where the light adjustable lens 120 can be attached to the IOL 110 at a proximal surface of the IOL 110. In this document, the terms "proximal" and "distal" are used in relation to the light incident from the pupil of the eye. Proximal indicates a position that is closer to the pupil. The shown embodiments differ in the manner the haptics 114-1 and 114-2, again, cumulatively haptics 114, are attached to the components of the CLA IOL 100.

**FIG. 2A** illustrates a CLA IOL 100, where the haptics 114 are attached to the IOL 110. For example, the haptics 114 can be molded together with the IOL 110, as is the case with many acrylic or acrylate IOLs, described above. These haptics 114 can be made of the same acrylic material as the IOL 110 itself, and can be molded in the same, single step as the IOL 110 itself. As described earlier, such integrated haptics 114 are easier to manufacture, are more reliably aligned with the IOL 110 and are less likely to separate from the IOL 110 during insertion.

**FIG. 2B** illustrates a CLA IOL 100 where the haptics 110 are attached to the light adjustable lens 120. Finally, **FIG. 2C** illustrates a CLA IOL 100 where the haptics 114 are attached to both the IOL 110 and the light adjustable lens 120 in a shared manner.

**FIG. 3** illustrates a CLA IOL 100, where the light adjustable lens 120 can be attached to the IOL 110 at a distal surface of the IOL 110. The light adjustable lens 120 - IOL 110 sequence of **FIGS. 2A-C,** and the IOL 110 - light adjustable lens 120 sequence of **FIG. 3** can both have their own advantages.

In some embodiments, the IOL 110 can be designed, or selected, to deliver the majority, or the entirety, of the intended optical power of the CLA IOL 100. In such embodiments, the light adjustable lens 120 can be designed only to provide the corrections and adjustments the surgeon anticipates may become necessary after the CLA IOL 100 settles in the eye with some unintended misalignment. Since the role of the light adjustable lens 120 in such embodiments is only to provide a correction of 1D-2D of optical power or cylinder, it can be a much thinner lens than in non-composite light adjustable IOLs, where all the optical power is generated by the light adjustable material. The CLA IOL embodiments that include only a corrective light adjustable lens 120 can therefore involve a much thinner light adjustable lens 120. The adjustment and lock-in of the light adjustable lens 120 in such a CLA IOL 100, described in relation to **FIG. 4,** therefore may require a smaller irradiation power, thereby increasing the safety of the overall light adjustment procedure.

The light adjustable lens 120 can be designed to provide a vision correction up to 2D, in other embodiments, only up to 1D. In some embodiments, either the IOL 110, or the light adjustable lens 120 can be a meniscus lens.

Concerning the chemical composition, in acrylate embodiments, the IOL 110 can include a monomer, a macromer, or a polymer, any one of which can include an acrylate, an alkyl acrylate, an aryl acrylate, a substituted aryl acrylate, a substituted alkyl acrylate, a vinyl, or copolymers combining alkyl acrylates and aryl acrylates. In some IOL 110s, the alkyl acrylate can include a methyl acrylate, an ethyl acrylate, a phenyl acrylate, or polymers and co-polymers thereof.

In some embodiments, the chemical composition of the IOL 110 can include a fractional mixing of the chemical composition of the light adjustable lens 120. Such an IOL 110 can include silicone-based monomers or macromers, forming polymers or copolymers with the acrylate, an alkyl acrylate, an aryl acrylate, a substituted aryl acrylate, a substituted alkyl acrylate, a vinyl, or copolymers combining alkyl acrylates and aryl acrylates.

In some embodiments, a monomer, a macromer, or a polymer of the IOL 110 can have a functional group that can include a hydroxy, amino, vinyl, mercapto, isocyanate, nitrile, carboxyl, or hydride. The functional group can be cationic, anionic or neutral.

According to the invention, the light adjustable lens 120 includes a first polymer matrix, and a refraction modulating composition, dispersed in the first polymer matrix, wherein the refraction modulating composition is capable

of a stimulus-induced polymerization that modulates a refraction of the light adjustable lens 120. The first polymer matrix can include a siloxane based polymer, formed from macromer and monomer building blocks with an alkyl group, or an aryl group.

In some embodiments of the composite light adjustable intraocular lens 100, the first polymer matrix can include a fractional mixing of at least one of an acrylate, an alkyl acrylate, an aryl acrylate, a substituted aryl acrylate, a substituted alkyl acrylate, a vinyl, and copolymers combining alkyl acrylates and aryl acrylates. These can form at least one of polymers and copolymers with compounds of the first polymer matrix.

The above embodiments, where the IOL 110 includes a fractional mixing of a material of the light adjustable lens 120, and where the light adjustable lens 120 includes a fractional mixing of a material of the IOL 110, can be formed to increase the compatibility of the materials of the lenses 110 and 120, thereby increasing the mechanical, physical and chemical robustness of the CLA IOL 100.

Embodiments of the light adjustable lens 120 can also include a photoinitiator, to absorb a refraction modulating illumination; to be activated upon the absorption of the illumination; and to initiate the polymerization of the refraction modulating compound. In some embodiments, the photoinitiator of the light adjustable intraocular lens 120 can also include an ultraviolet absorber.

Embodiments of light adjustable lenses 120 have been described in substantial detail in the commonly owned US Patent No: 6,450,642, to J.M. Jethmalani et al., entitled: "Lenses capable of post-fabrication power modification".

**FIG.** 4 illustrates four steps 101a-101d of a process of modifying a refraction property of the light adjustable lens 120 by illumination. Very briefly, in step 101a, the light adjustable lens 120 that includes a matrix and within it photosensitive macromers made from suitable materials, such as silicones, is illuminated by a lens adjusting light with a radial profile.

In step 101b, the exposure to the adjusting light causes the photosensitive macromers to polymerize with a radial profile, determined by the radial profile of the adjusting light.

In step 101c, the unpolymerized macromers diffuse to the central region where the photosensitive macromers photopolymerized previously. This causes a swelling of the light adjustable lens 120 in this central region. (In complementary processes, where the radial profile of the illuminating light in more intense towards the peripheral annulus of the light adjustable lens 120, the unpolymerized macromers diffuse outward to the peripheral annulus, causing the swelling of this peripheral annulus.)

Still in step 101c, the swelling can be followed by applying a lock-in light with an essentially uniform radial profile and greater intensity to polymerize all remaining macromers. In step 101d, this lock-in causes the light adjustable lens 120 to reach and to stabilize a shape that is swollen in its center, and therefore has a light-adjusted optical power. The above is only a very brief summary of the light adjustable lenses and their light adjustment procedure. A much more detailed explanation is provided in the US Patent No: 6,450,642, to J.M. Jethmalani et al..

In some embodiments, the IOL 110 and the light adjustable lens 120 are adapted to retain a chemical separation even after they are attached. This chemical separation can be achieved, e.g., by employing a refraction modulating composition in the light adjustable lens 120 that is not soluble in the materials of the IOL 110, and thus it does not diffuse into the IOL 110 from the light adjustable lens 120, in spite of the mobility of its constituent macromers in the first polymer matrix of the light adjustable lens 120 itself.

As mentioned before, one of the advantages of combining the IOL 110 that can be acrylic-based, with the light adjustable lens 120 that can be silicone-based is that an elastic constant of an acrylic IOL 110 can be softer than a corresponding elastic constant of a silicone light adjustable lens 120. In a CLA IOL 100, where the IOL 110 is considerably softer than the light adjustable lens 120, the "springiness" of the overall CLA IOL 100 can be considerably reduced relative to that of the light adjustable lens 120 alone. Such a CLA IOL 100 can be inserted with substantially improved control and predictability during cataract surgery, thus improving the surgical outcome.

As described in relation to **FIG. 4,** in some embodiments, the refraction properties of the light adjustable lens 120 are modified by applying an ultraviolet (UV) illumination. Safety considerations dictate that the applied UV illumination shall be prevented from reaching the retina of the eye, or at least the intensity of its transmitted component greatly attenuated. To this end, some embodiments of the CLA IOL 100 may contain UV absorbers. There are several different designs for including a UV absorber.

In some embodiments, the UV absorber can be related to the light adjustable lens 120. **FIG. 5** illustrates that in some designs, an ultraviolet absorbing layer 130 can be formed at a distal surface of the light adjustable lens 120. In other embodiments, an ultraviolet absorbing material can be dispersed throughout the light adjustable lens 120.

In other designs, the UV absorber can be related to the IOL 110. Since the UV light needs to reach the light adjustable lens 120 for the adjustment procedure, in such embodiments the light adjustable lens 120 can be attached to the IOL 110 at a proximal surface of the IOL 110, so that the UV absorber in the IOL 110 does not block the UV illumination from reaching the light adjustable lens 120. With such an arrangement, in some embodiments, an ultraviolet absorbing material can be dispersed throughout the IOL 110; in others, the CLA IOL 100 can include the ultraviolet absorbing layer 130. This ultraviolet absorbing layer 130 can be on a proximal or on a distal surface of the IOL 110, since either of these designs still places the ultraviolet absorbing layer 130 distal to the light adjustable lens 120.

In embodiments of the composite light adjustable intraocular lens 100, the light adjustable lens 120 can be attached to the IOL 110 by a variety of designs. In some cases, the light adjustable lens 120 can be attached to the IOL 110 by a chemical reaction, a thermal treatment, an illumination treatment, a polymerization process, a molding step, a curing step, a lathing step, a cryo-lathing step, a mechanical process, an application of an adhesive, or by any combination of these methods.

**FIG. 6** illustrates that some embodiments of the CLA IOL 100 can include an attachment structure 135 for attaching the light adjustable lens 120 to the IOL 110. This attachment structure 135 can include a cylinder, a ring, an open tub, into which an optical element can be inserted, or a clasp, among others. Such structures can have multiple advantages.

(a) For example, CLA IOLs 100 with an attachment structure 135 can be modular. This can be advantageous for pre-operative purposes, as a surgeon may need to keep a much smaller inventory. Once pre-operative diagnostics determines what IOL 110 needs to be paired with what light adjustable lens 120, the surgeon can select a separately stored IOL 110, and a separately stored light adjustable lens 120, and assemble the CLA IOL 100 by inserting the two selected lenses into the attachment structure 135.

(b) The modularity can be advantageous post-operatively as well. If at the end of the cataract surgery it is determined that for whatever reason, the IOL 110 was not selected optimally, if a non-modular CLA IOL 100 was used, then the surgeon needs to reopen the eye and remove the entire implanted CLA IOL 100, including its extended haptics 114. Such a full-IOL removal can pose substantial challenges and can lead to undesirable medical outcomes, such as broken haptic pieces.

In contrast, if a modular CLA IOL 100 was implanted, then, upon the reopening of the eye the surgeon does not need to remove the entire CLA IOL 100, only the non-optimal IOL 110, and exchange it with a better selected IOL 110. This procedure avoids the need to remove the entire CLA IOL 100, and thus reduces the risk of undesirable medical outcomes. Also, typically such replacement procedures may need a shorter incision, since only parts of the IOL are being replaced: another medical benefit.

(c) Finally, IOLs with taller structures have benefits in the context of reducing Posterior Capsule Opacification, or PCO. This will be described below in more detail in relation to **FIGS. 12A-B.** A CLA IOL 100 with an attachment structure 135 can be made as tall as desired by the surgeon.

In embodiments of the CLA IOL 100, the IOL 110 can be an advanced and complex IOL, such as a multifocal IOL, an aspheric IOL, a toric IOL, or a diffractive IOL. Such advanced IOLs offer vision corrections beyond the correction of the optical power alone. They can help reducing presbyopia, astigmatism, cylinder, or other types of aberrations. However, the performance of these advanced IOLs requires the placement of the IOL with higher than usual precision. If the implanted IOL ends up misplaced, or misaligned, at the end of cataract surgery or later, the vision improvements and benefits can be substantially inferior relative to the outcomes promised to the patient. The fact that such unintended misalignments and rotations happen in a notable percent of cataract surgeries is a key factor limiting the wider market acceptance of such advanced IOLs.

In contrast, if a CLA IOL 100 gets misplaced, misaligned, or rotated relative to the planned location, angle, or direction in the eye, the light adjustable lens 120 of the CLA IOL 100 can be adjusted to compensate this misalignment, or rotation. Therefore, CLA IOLs 100 have the potential to deliver the promised vision improvements to the patients reliably. This benefit of the CLA IOLs 100 can start a fast expansion of the market acceptance and the market share of the advanced IOLs.

In some other embodiments, the insertion of the embodiments of **FIG. 6** can be eased by making the attachment structure 135 a fluid-fillable structure instead of a hard structure. Such a fluid-fillable attachment structure135 can be inserted into the eye in its unfilled form and then filled up with liquid only after insertion. In some embodiments, a UV absorbing layer 130 can be provided at the distal surface of the light adjustable lens 120.

**FIGS. 7A-C, FIGS. 8A-C** and **FIG. 9** illustrate the above general considerations on a CLA IOL 100 that includes a toric IOL 110, aimed at correcting a cylinder in an eye.

**FIG. 7A** illustrates a surgical situation where, to compensate a cylinder in an eye, a surgeon decided to implant a CLA IOL 100 with a toric IOL 110, whose target toric axis 202 was planned to be oriented in the indicated direction - for simplicity and clarity, chosen as straight up in the plane of **FIG. 7A.** Toric IOLs often include axis markers 203 to indicate the direction of the toric axis for the surgeon.

**FIG. 7B** illustrates that, after the end of the cataract surgery and the closing of the incisions, the implanted CLA IOL 100 may have rotated for a variety of reasons, so that the implanted rotated toric axis 204 of the implanted CLA IOL 100 makes an unintended rotational angle $\alpha$ with the target toric axis 202.

**FIG. 7C** illustrates that the surgeon can devise and carry out an illumination procedure on the light adjustable lens 120 of the CLA IOL 100 to form a counter-rotating toric pattern 206, thereby causing a counter-rotation of the overall toric axis, so that the corrected toric axis 208 after the light adjustment procedure ends up pointing in the same direction as the

originally planned target toric axis 202.

**FIG. 8A** illustrates the same procedure on the level of the cylinder patterns 212-218. The surgeon in the pre-surgical planning phase of the cataract surgery may have decided that the cylinder vision problem of the patient shall be cured by implanting a CLA IOL 100 with a toric IOL 110, that has a target cylinder pattern 212, oriented as shown. However, after the implantation, the CLA IOL 100 may have unintentionally rotated to an implanted rotated cylinder 214. Such a misaligned, rotated cylinder 214 provides a much-reduced vision improvement, as explained previously. As the rotational angle grows, the implanted rotated cylinder 214 can even turn into a net negative effect, being more a nuisance and disorientation than a benefit for the patient.

To compensate this unwanted medical outcome, the surgeon can carry out a post-surgical diagnostic procedure to determine a corrective counter-rotating cylinder 216, the implementation of which can correct the unintended and unwanted rotation of the CLA IOL 100. As shown, the surgeon can perform a light adjustment procedure of the light adjustable lens 120 of the CLA IOL 100 in order to create the counter-rotating cylinder 216 in the light adjustable lens 120. The superposition of the implanted rotated cylinder 214 and the counter-rotating cylinder 216 can sum up into a shape of the light adjustable lens having a corrected cylinder 218, whose direction is aligned with the direction of the originally planned target cylinder 212. These steps are analogous to the steps of **FIGS. 7A-C,** described previously.

**FIG. 8B** illustrates the same procedure in a geometric language, where the cylinder patterns are represented by corresponding vectors. The directions of the vectors are indicative of the directions of the represented cylinders, and the magnitudes of the vectors can represent the strength, curvature, or diopters of the cylinders. The target toric vector 222 represents the target cylinder 212, and the implanted rotated toric vector 224 represents the implanted rotated cylinder 214 of the CLA IOL 100 after implantation. As before, the surgeon post-operatively can determine the counter-rotating toric vector 226, representing the counter-rotating cylinder 216, necessary to correct the unintended post-surgical rotation of the toric IOL 110. When the surgeon performs the light adjustment procedure to adjust the light adjustable lens with the counter-rotating toric vector 226, the superposition of the implanted rotated toric vector 224 and the counter-rotating toric vector 226 restores the corrected toric vector 228 to have the same direction and magnitude as the target toric vector 222.

**FIG. 8C** illustrates in the language of the vector representation that there can be different ways to bring about the necessary correction. For example, the correctional pattern can include a reductional toric vector 227 that reduces, or even eliminates, the implanted rotated toric vector 224. The counter-rotating toric vector 226 can then be chosen to rotate the remaining portion of vector 224 (that is equal to the sum of the vectors 224 and 227) into the corrected toric vector 228.

In a demonstrative example, in an embodiment of the CLA IOL 100 that includes a toric IOL 110 for correcting a cylinder greater than 2D, the light adjustable lens 120 can be adapted to be able to correct a cylinder up to 2D. For example, if the toric IOL 110 was intended to correct a 6D cylinder, but the toric axis was rotated by 10 degrees, this translates into a 30% reduction of efficiency, as described earlier, providing a net 4D cylinder improvement for the patient. However, the surgeon can perform a light adjustment procedure on the light adjustable lens 120 to correct the 2D cylinder that was lost to the unintended rotation, thereby restoring the full 6D cylinder promised to the patient.

**FIG. 9** illustrates the steps of a corresponding method 230 of adjusting an implanted composite light adjustable intraocular lens 100 in more general terms. The method 230 can include the following steps.

231 - Planning a targeted optical outcome of an implantation of a composite light adjustable intraocular lens into an eye.

232 - Implanting the composite light adjustable intraocular lens into the eye.

233 - Performing a diagnostic measurement to evaluate an implanted optical outcome of the implantation.

234 - Determining a correction based on a comparison of the planned optical outcome and the implanted optical outcome.

235 - Applying a stimulus to adjust an optical characteristic of the composite light adjustable intraocular lens to induce the determined correction.

In the procedure described in relation to **FIGS. 7A-C** and **FIGS. 8A-C,** the method 230 can be adapted for a case where the targeted optical outcome is the target cylinder 202/212/222; the implanted optical outcome is an implanted rotated cylinder 204/214/224; and the determined correction is a counter-rotating cylinder 206/216/226. These steps can adjust the implanted rotated cylinder 204/214/224 into the corrected cylinder 208/218/228, that is closely related to the target cylinder 202/212/222.

Next, **FIGS. 10-11** illustrate an embodiment of the CLA IOL 100 that provides the additional medical benefit of chromatic aberration reduction. This embodiment is developed starting from the observation that the optical system of the eye, its main constituents being the cornea and the lens, exhibits a chromatic dispersion, as the effective index of refraction

$n_e$ of the involved eye tissues depend on the wavelength of the light: $n_e=n_e(\lambda)$. It has been found that the derivative of $n_e(\lambda)$ is typically negative: $\partial n_e/\partial\lambda<0$. Therefore, the optical power $P_e$ of the eye, proportional to ($n_e$-1), also has a negative derivative with respect to the wavelength: $\partial P_e/\partial\lambda<0$. Even for healthy persons with 20/20 vision, this chromatic dispersion of the eye tissues causes the short wavelength ("blue") components of an image focused and imaged proximal the retina, while the long wavelength ("red") components focused distal to the retina, thereby causing some degree of blurring and image quality deterioration. This blurring of the color-components of the image is often referred to as chromatic aberration.

Our brain learned to accept a limited degree of this chromatic aberration. Nevertheless, cataract surgery has the opportunity to provide an additional medical benefit by implanting chromatic aberration-compensating IOLs that compensate the eye's own chromatic aberration and image all wavelength components to the retina, thereby reducing the chromatic aberration and sharpening the vision.

The dependence of the index of refraction on the wavelength is often characterized by the Abbe number, defined as $V=(n_D-1)/(n_F-n_C)$, where $n_D$, $n_F$, and $n_C$ are the indices of refraction at the Fraunhofer D, F, and C spectral lines at 589, 486, and 656 nm, respectively. Most Abbe numbers are in the 20-90 range. For corneal and lens tissue, the Abbe number is in the 50-60 range. The optical power $P_l$ of the intraocular lens depends on the index of refraction $n_l(\lambda)$ through the lensmaker's equation: $P_l=(n_l-1)(1/R_1-1/R_2)$, where $R_1$ and $R_2$ are the radii of curvature of the two surfaces of the intraocular lens. Therefore, the $\lambda$ dependence of $n_l$ makes the intraocular lens optical power $P_l$ also depend on the wavelength $\lambda$: $P_l=P_l(\lambda)$. It is noted that this dependence involves the sign of the optical power of the lens. For positive optical power lenses, the typically negative $\partial n_l/\partial\lambda<0$ translates to a negative $\partial P_l/\partial\lambda<0$, whereas for negative optical powers, the negative $\partial n_l/\partial\lambda<0$ translates to a positive $\partial P_l/\partial\lambda>0$.

With these introductory remarks, an intraocular lens can compensate chromatic aberrations, if the wavelength derivative of its optical power compensates the negative wavelength derivative of the eye optical power, so that $\partial P_l/\partial\lambda + \partial P_e/\partial\lambda \approx 0$. In other words, $\partial P_l/\partial\lambda \approx -\partial P_e/\partial\lambda>0$.

Now, since regular (non-diffractive) intraocular lenses deliver a positive optical power $P_l$ of about 20D, in the light of the introductory remarks, their $\partial P_l/\partial\lambda$ is negative, and thus they are unable to compensate the eye's own chromatic aberrations, because $\partial P_e/\partial\lambda$ is also negative.

However, embodiments of the CLA IOL 100 are made of two different lenses, the IOL 110, and the light adjustable lens 120. Such two-lens designs introduce a genuinely new possibility. One of the lenses of the CLA IOL 100 can have a negative optical power and thus a strongly positive $\partial P/\partial\lambda>0$, so that the combined, two-lens CLA IOL 100 can compensate the chromatic aberration of the eye optical system, while the combined optical powers of the two lenses can still perform the primary function of the intraocular lens, to deliver an about constant 20D. In formulae, the first lens optical power $P_{l,1}$ and the second lens optical power $P_{l,2}$ of a two-lens CLA IOL 100 can simultaneously satisfy the following two relations:

$$P_{l,1}+P_{l,2}=20D, \qquad (1)$$

$$\partial P_{l,1}/\partial\lambda + \partial P_{l,2}/\partial\lambda \approx -\partial P_e/\partial\lambda > 0 \qquad (2)$$

In some detail, **FIGS. 10A-B** show embodiments of the CLA IOL 100 which deliver such reduced chromatic aberrations. Traditionally, in such composite lenses, the negative optical power lens is often referred to as a "flint", the positive optical power lens as a "crown". If the composite lens itself exhibits near zero chromatic aberration, then the CLA IOL 100 can be called an "achromat". If the composite lens makes a larger assembly, such as the CLA IOL 100 plus the eye, exhibit near zero chromatic aberration, then the CLA IOL 100 can be called an "achromator".

**FIG. 10A** shows an embodiment where the IOL 110, having a negative optical power $P_{IOL}<0$, is the flint, and the light adjustable lens (LAL) 120, having a positive optical power $P_{LAL}>0$, is the crown. **FIG. 10B** illustrates an opposite embodiment, where the IOL 110 has a positive power $P_{IOL}>0$, and the light adjustable lens 120 has a negative power $P_{LAL}<0$.

The magnitude of $\partial n/\partial\lambda$, $|\partial n/\partial\lambda|$ is relatively high for PMMA, while $|\partial n/\partial\lambda|$ is typically low for silicone. Therefore, CLA IOLs 100 embodiments with the design of **FIG. 10A,** where the negative power IOL 110 is made of PMMA, or other acrylates or analogs, and the positive power light adjustable lens 120 is made of silicone, can reduce the chromatic aberration efficiently. In this embodiment, the high $|\partial n/\partial\lambda|$ PMMA IOL 110 can be given a low and negative optical power, such as $P_{IOL} \approx$ -10D, while the low $|\partial n/\partial\lambda|$ silicone LAL 120 can be given a high optical power, $P_{LAL} \approx$ +30D, so that the combined optical power of the entire CLA IOL 100 is:

$$P_{IOL} + P_{LAL} \approx +20D, \qquad (3)$$

while at the same time the CLA IOL 100 is still capable of compensating the chromatic aberration of the eye:

$$\partial P_{IOL}/\partial\lambda + \partial P_{LAL}/\partial\lambda \approx -\partial P_e/\partial\lambda \quad (4)$$

Such a CLA IOL 100 can deliver an overall optical power of about 20D, while the combined wavelength derivatives of the optical powers of the IOL 110 and the LAL 120 can largely compensate the chromatic aberrations of the eye optical system, thereby substantially reducing the overall chromatic aberration of the eye after the implantation of the CLA IOL 100. (Here, the "eye optical system" primarily refers to the cornea, as the crystalline lens has been removed by the cataract surgery.)

**FIG. 11** illustrates the above concepts in the language of the chromatic shifts. The chromatic shift characterizes the distance of the image from the target/image plane (in the case of the eye, from the retina), expressed in diopters. A negative chromatic shift represents that the image was formed proximal, in front of the retina, whereas a positive chromatic shift that the image was formed distal, behind the retina. Thus, the chromatic shift increasing with the wavelength represents that the optical power decreases with the wavelength: $\partial P/\partial\lambda<0$.

**FIG. 11** shows that the natural eye optical system alone has an increasing chromatic shift, consistent with $\partial P_e/\partial\lambda<0$. A one component IOL 110 alone typically also has an increasing chromatic shift, consistent with $\partial P_{IOL}/\partial\lambda<0$. The dashed "composite light adjustable IOL" line indicates that if a chromatic aberration-compensating embodiment of the CLA IOL 100 is implanted into the eye, then the combined CLA IOL 100 plus eye system can exhibit minimal chromatic shift and chromatic aberration.

Accordingly, in embodiments of the CLA IOL 100, the IOL 110 has an IOL chromatic shift variation; the light adjustable lens 120 has a light adjustable lens chromatic shift variation; an eye, with a crystalline lens removed, has an eye chromatic shift variation; and a chromatic shift variation of the eye, with the composite light adjustable intraocular lens 100 implanted, can be less than a chromatic shift variation of the eye with the crystalline lens in place, wherein the chromatic shift variation is defined from a difference of a chromatic shift at 450 nm and at 650 nm.

In embodiments of the CLA IOL 100, an optical power of the IOL 110 can be negative; an optical power of the light adjustable lens 120 can be positive; and the chromatic shift variation of the eye, with the composite light adjustable intraocular lens implanted, can be less than 0.5D. In other embodiments, this chromatic shift variation can be less than 0.2D. In eyes with such achromator CLA IOLs 100 implanted, the blurriness of the image, caused by the chromatic dispersion, can be substantially smaller than that of the natural eye, thereby sharpening the vision in an additional aspect: a clear further medical benefit.

Ideas about achromator IOLs with related aspects has been proposed by E.J. Fernandez and P. Artal in an article entitled: "Achromatic doublet intraocular lens for full aberration correction", at p. 2396, vol. 8 (2017) of the Biomedical Optics Express. While instructive in some aspects, this paper did not discuss, among others, aspects of light adjustability of the involved IOLs. Adapting the technology of this paper for light adjustable lenses involves further advanced concepts.

**FIGS. 12A-B** illustrate a further medical benefit of CLA IOLs 100, especially those where either the IOL 110, or the light adjustable lens 120 has a negative optical power, and therefore has an unusually tall side 142 and a sharp IOL edge 144. In such embodiments, the sharp IOL edge 144 may be pushed against a capsular bag 15 of the eye in which it was inserted, by a force larger than the force pushing one component intraocular lenses.

This enhanced force can have the following notable medical benefit. Posterior capsule opacification, PCO, is one of the well-known negative outcomes, or complications, of cataract surgery. PCO results from the growth and abnormal proliferation of lens epithelial cells (LECs) on the posterior capsule. Most PCOs are fibrous, or pearl-like, or a combination of both. Clinically, PCO can be detected as a wrinkling on the posterior capsule, for example. The development of PCO often involves three basic phenomena: proliferation, migration and differentiation of residual LECs.

While various pharmaceutical solutions have been developed to mitigate PCO, forming a sharp mechanical barrier in contact with the capsular bag 15 was also shown to reduce PCO. Such a barrier suppresses the fibrous growth and reduces LEC migration, thereby reducing PCO.

In embodiments of the CLA IOL 100, the sharp IOL edge 144 is pushed against the capsular bag 15 with unusually high force because the flint lens of the achromat has an unusually tall side 142 since it has negative optical power and thus its side is taller than its center. For this reason, achromat embodiments of the CLA IOL 100 exhibit the additional medical benefit of PCO reduction.

**FIG. 12A** and **FIG. 12B** illustrate that there can be several combinations and designs of the CLA IOL 100 that press the capsular bag 15 with higher than usual force. For example, the sequence of the IOL 110 and the light adjustable lens 120 can be reversed. In other embodiments, the materials of the flint and the crown can be exchanged. CLA IOLs 100 that have a distal crown lens, i.e. a crown lens that is closer to the retina can exhibit advantageously lower aberrations, as the distalmost surface of such CLA IOLs 100 is closest in shape to the shape of the retina. In contrast, if the flint is closer to the retina, then the distalmost surface is substantially different from the surface of the retina, giving rise to higher aberrations.

Yet another medical benefit of these CLA IOLs 100 with tall sides is that the higher pressing forces induce higher capsular bag tensions. This higher capsular tension tends to stabilize the location and the axis of the CLA IOL 100 better

than the lower capsular tension induced by the flat regular IOLs, thereby preventing the CLA IOL 100 from tilting, or otherwise getting misaligned.

The taller IOL side 142 may necessitate the formation of a larger, or longer, surgical incision. This, in turn, may induce an unintentional astigmatism after the cataract surgery. However, since the light adjustable lens 120 can be adjusted after the surgery, in embodiments of the CLA IOL 100, this astigmatism can be compensated and eliminated efficiently by applying an astigmatism-compensating light adjustment procedure to the light adjustable lens 120.

**FIG. 13** illustrates the composite light adjustable intraocular lens 100 according to the invention that comprise an acrylic intraocular insert 110', a silicone-based light adjustable lens 120, attached to the acrylic intraocular insert 110' with an adhesion promoter 300; and haptics 114-1 and 114-2. The adhesion promoter 300 includes a first orthogonal functional group, configured to bond with an acrylic component of the acrylic intraocular insert 110'; and a second orthogonal functional group, configured to bond with a silicone component of the silicone-based light adjustable lens 120, as described below in detail. For simplicity, in parts of the description and in the Figures, the silicone-based light adjustable lens 120 is sometimes abbreviated as light adjustable lens 120, or LAL 120.

The acrylic intraocular insert 110' can include the intraocular lens (IOL) 110 with an optical power and can be thought of as an embodiment of the IOL 110. In some cases, the acrylic intraocular insert 110' can include a carrier with approximately zero optical power. And in reverse, the intraocular lens (IOL) 110 can be thought of as an embodiment of the acrylic intraocular insert 110', with, or without an optical power.

According to the invention the adhesion promoter 300 ensures that the two main constituents of the composite light adjustable IOL 100, the acrylic intraocular insert 110' and the silicone-based light adjustable lens 120 are chemically bonded together and do not peel apart after implantation and the light adjustment procedure.

Attaching an acrylic IOL to a silicone frame around its edges, or to a silicone biasing element by chemical means for presbyopic applications has been described before. However, there are at least the following differences between those IOLs and the presently described CLA IOL 100.

(1) The optical, or viewing, elements in those IOLs have unchanging, fixed shapes. Therefore, the strains and tensions at the acrylic-silicone joints can be minimized by a suitable fabrication process. Also, IOLs with underperforming joints can be discarded as part of the quality control during the fabrication process. This is in contrast to the embodiments of the CLA IOL 100, where the light adjustment procedure changes the shape of the silicone-based light adjustable lens 120 after the implantation, while leaving the shape of the acrylic intraocular insert 100' essentially unchanged, as illustrated in detail in **FIGS. 14A-C.** In these CLA IOLs 100, the light adjustment procedure induces shear and stress to the silicone-acrylic joint after the implantation, possibly even inducing the two elements to peel away from each other. Since the magnitude of the adjustment varies from patient to patient, the light induced stresses and tensions cannot be minimized by a suitable fabrication process prior to implantation. Instead, the silicone-acrylic joint needs to be stress-tolerant, resisting the light-induced tensions to the necessary degree, including resisting the tendencies of the silicone-based light adjustable lens 120 peeling away from the acrylic intraocular insert 110' as a result of the light adjustment. This is a particularly demanding expectation, as implanted CLA IOLs 100 with peeled-apart components cannot be discarded.

(2) In most of the previously described IOLs, the silicone forms a frame, or a biasing element, or is positioned around the edges of the acrylic IOL. In such IOLs, the silicone-acrylate interface is not within the optical path, and therefore there is not much of a requirement for the silicone-acrylic joint to not distort the imaging quality. In contrast, in embodiments of the CLA IOL 100, the silicone-acrylic interface is within the optical path, and thus the entire interface is expected to transmit light without distortion in spite of the light adjustment procedure inducing tensions and strains at the silicone-acrylic interface. The above two considerations illustrate marked differences between previous designs and embodiments of the CLA IOL 100.

**FIGS. 14A-C** illustrate point (1) above regarding the difference of the here-described CLA IOLs 100 from previous systems, driven by the light adjustability of the silicone-based LAL 120. **FIG. 14A** illustrates the application of the refraction modulating illumination to the CLA IOL 100. **FIG. 14B** illustrates that in response to this illumination, the silicone-based LAL 120 of the CLA IOL 100 changes its shape. The shown case illustrates a light adjustment procedure that increases the optical power of the silicone-based LAL 120, wherein the radius of curvature of the frontal surface decreases, while the radius of curvature of the back-surface often increases. In light adjustment procedures that reduce the optical power, the opposite changes of curvature are induced. **FIG. 14C** illustrates a close-up of a portion of the interface between the acrylic intraocular insert 110' and the silicone-based LAL 120. The refraction modulating illumination causes a curvature change and lateral sheer 111 of the silicone-based LAL 120, peeling it away from the acrylic intraocular insert 110', thus potentially inducing a separation 112. Therefore, in contrast to IOLs with fixed-shape elements, the refraction modulating illumination unavoidably induces tension and strain at the interface of the acrylic intraocular insert 110' and the silicone-based LAL 120. This strain and tension are induced only after the fabrication process and after the implantation, thus cannot be eliminated by changes in fabrication. Thus, CLA IOLs 100 require an adhesion promoter 300 that chemically bonds the two surfaces

together with sufficient strength to prevent the separation of the acrylic intraocular insert 110' and the silicone-based LAL 120, in spite of the tensions and strain induced by the shape change caused by the refraction modulating illumination.

**FIGS. 15A-B** illustrate that, as before, in some embodiments of the composite light adjustable intraocular lens 100, or CLA IOL 100, the haptics 114-1 and 114-2 can be attached to the acrylic intraocular insert 110'; in others the haptics 114-1/114-2 can be part of the acrylic intraocular insert 110'; in yet others, the haptics 114-1/114-2 can be attached to the silicone-based light adjustable lens 120; and finally in some CLA IOLs 100 the haptics 114-1/114-2 can be attached to both the acrylic intraocular insert 110' and the silicone-based light adjustable lens 120.

**FIGS. 13-17** illustrate embodiments of the CLA IOL 100, where the silicone-based light adjustable lens 120 is attached to the acrylic intraocular insert 110' proximally, i.e. positioned towards the cornea of the eye.

Just like IOLs 110, embodiments of the acrylic intraocular insert 110' can comprise at least one of a monomer, a macromer, an oligomer, and a polymer, selected from the group consisting of an acrylate, an alkyl acrylate, an aryl acrylate, a substituted aryl acrylate, a substituted alkyl acrylate, a halogen substituted acrylate, a halogen substituted methacrylate, an acrylic ester or acrylic acid, an acrylamide, a vinyl, and copolymers combining alkyl acrylates and aryl acrylates. For some CLA IOLs 100, the aforementioned monomers can be a methyl acrylate, an ethyl acrylate, an ethyl hexyl acrylate, a phenyl acrylate, an ethyl methacrylate, a trifluoro ethyl acrylate, a trifluoro ethyl methacrylate, an n-butyl acrylate, a hydroxy ethyl acrylate, a hydroxy methyl acrylate, an n-vinyl pyrrolidone, a phenoxyethyl acrylate, or polymers or co-polymers thereof.

Additionally, crosslinkers with corresponding bis- or multi-functionality can be present to aid the polymerization. For some CLA IOLs 100, the crosslinker can be ethylene glycol dimethacrylate, tetraethylene glycol dimethacrylate, propylene glycoldimethacrylate and propylene glycoldiethacrylate. Furthermore, the crosslinked network can be induced by thermal, UV-initiated, or catalyst-promoted reactions. For some CLA IOLs 100, thermal initiators can be 2,2-azobis(2,4-dimethylpentanitrile), 2,2-azobis(2,4-dimethylbutanenitrile), azobisisobutyro-nitrile, azobisisopropionitrile, or azobisisomethylpropionitrile. For some CLA IOLs 100, photo initiators can be benzophenones, benzoin alkyl ethers, benzil ketals, phosphine oxides, acyl oxime esters, acetophenones or acetophenone derivatives.

As described earlier in relation to **FIG. 4,** the silicone-based light adjustable lens 120 can include a first polymer matrix and a refraction modulating composition, dispersed in the first polymer matrix, wherein the refraction modulating composition is capable of stimulus-induced polymerization that modulates a refraction of the silicone-based light adjustable lens 120. The first polymer matrix can include a siloxane-based polymer, formed from macromer and monomer building blocks with at least one of an alkyl group and an aryl group.

In addition, the CLA IOL 100 can include a photoinitiator, configured to be activated upon absorbing a refraction modulating illumination; and to initiate the stimulus-induced polymerization of the refraction modulating compound. To provide protection and safety. CLA IOLs 100 can include an ultraviolet absorber.

**FIGS. 16A-B** illustrate different ways of incorporating the adhesion promoter 300 into the CLA IOL 100. **FIG. 16A** illustrates that in some CLA IOLs 100, the adhesion promoter 300 can be dispersed in the acrylic intraocular insert 110'. **FIG. 16B** illustrates that in some CLA IOLs 100 the adhesion promoter 300 can be dispersed in an adhesion layer 310 between the acrylic intraocular insert 110' and the silicone-based light adjustable lens 120. Finally, in some CLA IOLs 100, the adhesion promoter 300 can be dispersed in the silicone-based light adjustable lens 120. In some embodiments, the adhesion promoter 300 can be dispersed in some combination of the embodiments of **FIGS. 17A-C.**

**FIGS. 17A-C** illustrate these same embodiments of inclusion in a somewhat different manner. **FIG. 17A** illustrates the embodiments of **FIG. 16A,** where the adhesion promoter 300 is primarily dispersed in the acrylic intraocular insert 110', bonded to the silicone-based LAL 120 with silicon-carbon covalent bonds 320, and bonded to acrylates of the acrylic intraocular insert 110' with bonds 322. The circles are schematic representations of the first orthogonal functional group configured to bond with an acrylic component of the acrylic intraocular insert 110' with bonds 322. The triangles are schematic representations of the second orthogonal functional group, configured to configured to bond with a silicone component of the silicone-based light adjustable lens 120 with covalent bonds 320. Both orthogonal functional groups, schematically represented by the circles and the triangles, are selected to uniquely bond with their complementary counterparts. **FIG. 17B** illustrates the embodiments of **FIG. 16B,** where the adhesion promoter 300 is primarily dispersed in the adhesion layer 310, bonded to the silicone-based LAL 120 with covalent chemical bonds 320, and bonded to the acrylates of the acrylic intraocular insert 110' with bonds 322. **FIG. 17C** illustrates the embodiments where the adhesion promoter 300 is primarily dispersed in the silicone-based LAL 120, bonded to the silicone-based LAL 120 with covalent chemical bonds 320, and bonded to the acrylates of the acrylic intraocular insert 110' with bonds 322.

Next, embodiments of the adhesion promoter 300 will be described in detail. As mentioned above, the adhesion promoter 300 contains the two orthogonal functional groups that can independently participate in polymerization using their unique chemistries: the first orthogonal functional group, configured to bond with an acrylic component of the acrylic intraocular insert 110', and the second orthogonal functional group, configured to bond with a silicone component of the silicone-based light adjustable lens 120. In some embodiments of the CLA IOL 100, the adhesion promoter 300 can have structure (1) as follows.

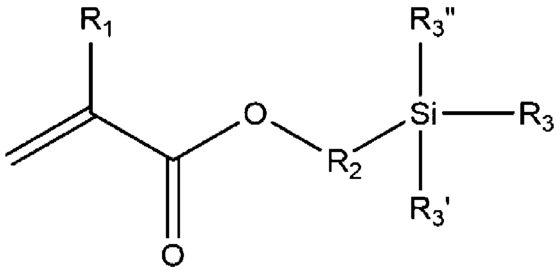

(1)

wherein

at least one of R3, R3' and R3" is a vinyl dialkylsiloxy pendant group with the structure (2)

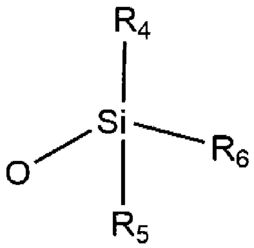

(2)

the remaining of R3, R3' and R3" are independently selected from the group consisting of C1-C10 pendant alkyl groups, such as methyl, ethyl, n-propyl, isopropyl, cyclopropyl, n-butyl, sec-butyl, t-butyl, cyclobutyl, or methylcyclopropyl;
the first orthogonal functional group is the functional group to the left of $R_2$;
the second orthogonal functional group is $R_6$;
$R_1$ is selected from the group consisting of a hydrogen, a monovalent hydrocarbon group, and a substituted C1-C12 alkyl, wherein the alkyl can be methyl, ethyl, n-propyl, isopropyl, cyclopropyl, n-butyl, sec-butyl, t-butyl, cyclobutyl, or methylcyclopropyl;
$R_2$ is an alkyl spacer with 1-10 carbon atoms, such as (-CH2)n, where n=1 through 10;
$R_4$ and $R_5$ are independently selected from the group consisting of C1-C10 pendant alkyl groups, such as methyl, ethyl, n-propyl, isopropyl, cyclopropyl, n-butyl, sec-butyl, t-butyl, cyclobutyl, or methylcyclopropyl; and
$R_6$ is one of a vinyl group, a vinyloxy group, an allyl group, an allyloxy group, and a group with a carbon chain C1-C10.

In some embodiments of the CLA IOL 100, R1 is methyl, R2 is propyl, R3, R3' and R3" each is vinyl dialkylsiloxy, R4 and R5 each is a C1-10 alkyl chain, and R6 is vinyl.

In some embodiments, R2 can be a chain, in others, a branched, or a cyclic isomer of an alkyl spacer, such as cyclopentyl. Further, R2 can be a substituted vinyl aryl. Optionally, R2 may also have a substituted aromatic group such as a substituted phenyl or a substituted naphthyl.

The first orthogonal functional group to the left of R2 forms the covalent bond 322 with the acrylic intraocular insert 110'. The second orthogonal functional group is R6 that forms the covalent bond 320 with the silicone-based LAL 120 via the double bond interacting with silicone hydride to form a new covalent silicone carbon bond 320.

Adhesion promoters 300 with more double bonds form more covalent bonds 320 with the silicone-based LAL 120. The number of available double bonds can be increased by selecting two or all three of R3, R3' and R3" to be a vinyl dialkylsiloxy pendant group, each containing an R6 with a double bond. The resulting adhesion promoter 300 is shown in structure (3).

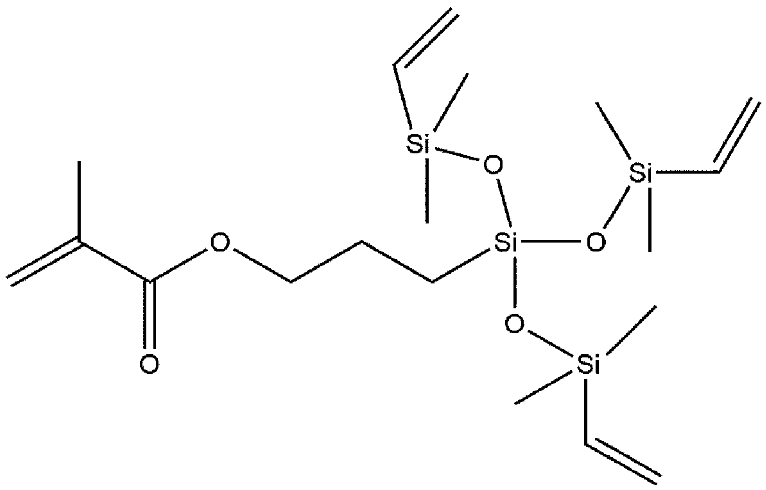

(3)

This embodiment of the adhesion promoter 300 is called methacryloxypropyltris (vinyldimethylsiloxy)silane, with structure (3), where R1 is methyl, R2 is propyl, the R3, R3' and R3" are vinyldimethilsiloxy, R4 and R5 are methyl, and R6 is vinyl. This structure (3) is a suitable an adhesion promoter 300, since it contains 3 vinyl R6s, each with a double bond, and thus is capable of bonding to the silicone-based LAL 120 with multiplied strength, which is promising for preventing the peeling between the acrylic intraocular insert 110' and the silicone-based LAL 120, and the optical distortions at their interface, as discussed above.

The overall strength of the adhesion between the acrylic intraocular insert 110' and the silicone-based LAL 120, generated by the adhesion promoter 300 depends on the strength and number of the covalent bonds 320 per individual adhesion promoter molecule, as well as on the concentration of these molecules. It has been found that a concentration of the methacryloxypropyltris(vinyldimethylsiloxy)silane as adhesion promoter 300 above 5 weight% dispersed in the acrylic intraocular insert 110' was sufficient (1) to prevent peeling between the acrylic intraocular insert 110' and the silicone-based LAL 120 even after the refraction modulating illumination, and (2) to avoid the generation of optical distortions by the acrylic intraocular insert 110' -- silicone-based LAL 120 interface. CLA IOLs 100 with a concentration above 10 weight% performed particularly well. For adhesion promoters 300 with structure (1) but where only one of the R3 groups was a vinyl dialkylsiloxy pendant group, a concentration higher than 10 weight% was found to provide sufficient quality bonding.

Structure (3) can be viewed as a monomer unit within the formulation of acrylic intraocular insert 110', where it can act as an adhesion promoter 300. In related embodiments, the corresponding building block can be

methacryloxypropyldi(vinyldimethylsiloxy)methylsilane,
methacryloxypropyl(vinyldimethylsiloxy)dimethylsilane,
acryloxypropyltris(vinyldimethylsiloxy)silane,
methacryloxybutyltris(vinyldimethylsiloxy)silane,
acryloxybutyltris(vinyldimethylsiloxy)silane,
acryloxypropyldi(vinyldimethylsiloxy)methylsilane,
methacryloxybutyldi(vinyldimethylsiloxy)methylsilane,
acryloxybutyldi(vinyldimethylsiloxy)methylsilane,
acryloxypropyl(vinyldimethylsiloxy)dimethylsilane,
methacryloxybutyl(vinyldimethylsiloxy)dimethylsilane,
acryloxybutyl(vinyldimethylsiloxy)dimethylsilane,
styrylmethyltris(vinyldimethylsiloxy)silane,
styrylethyltris(vinyldimethylsiloxy)silane,
styrylmethyltrisdi(vinyldimethylsiloxy)silane,
styrylethyldi(vinyldimethylsiloxy) methylsilane,
styrylethyl(vinyldimethylsiloxy)dimethylsilane, and
styrylethyl(vinyldimethylsiloxy)dimethylsilane, to name a few.

This unit can be coupled to the silicone-based LAL 120 by covalent bonds 320 between one or more silicon atoms of the silicone-based LAL 120 and one or more carbon atoms of the adhesion promoter 300, typically through its R6 group.

In some CLA IOLs 100, the covalent bonds are created via a hydrosilation reaction between the vinyl group of the vinyldialkylsiloxy group and an Si-H group of the silicone-based light adjustable lens 120. Adhesion promoters 300 with more branches (n>1) have more double bonded second orthogonal functional groups, and thus can provide stronger

bonding to the silicone-based light adjustable lens 120, as mentioned in relation to structure (3) above.

**FIGS. 18A-B** illustrates that in some CLA IOLs 100 the silicone-based light adjustable lens 120 is attached to the acrylic intraocular insert 110' at a proximal surface of the acrylic intraocular insert 110'. In these CLA IOLs 100 the acrylic intraocular insert 110' can include an ultraviolet absorbing material dispersed throughout the acrylic intraocular insert 110', or an ultraviolet (UV) absorbing layer 340. This UV absorbing layer 340 can be at a proximal surface of the acrylic intraocular insert 110', as in **FIG. 18A,** or at a distal surface of the acrylic intraocular insert 110', as in **FIG. 18B.** The CLA IOL 100 of **FIG. 18A** can be equivalently characterized as the ultraviolet absorbing layer 340 being formed at a distal surface of the silicone-based light adjustable lens 120. All these embodiments can be helpful to further increase the retinal safety of the refraction modulation illumination.

In CLA IOLs 100, the silicone-based light adjustable lens 120 can be attached to the acrylic intraocular insert 110' by at least one of a chemical reaction, a thermal treatment, an illumination treatment, a polymerization process, a molding step, a curing step, a lathing step, a cryo-lathing step, a mechanical process, an application of an adhesive, and by a combination thereof.

**FIGS. 19A-B** illustrate that the acrylic intraocular insert 110' having an optical power can include a diffractive structure 350 to induce this optical power, as was mentioned before. This diffractive structure 350 can be at the distal surface of the acrylic intraocular insert 110', as shown. In other cases, the diffractive structure 350 can be at the proximal surface of the acrylic intraocular insert 110', facing the silicone-based LAL 120. This latter design reduces the halo and glare, characteristic of diffractive IOLs.

In some embodiments of the composite light adjustable IOL 100, the acrylic intraocular insert 110' can be a toric acrylic intraocular insert 110'. This toric acrylic intraocular insert 110' can have an optical power in some embodiments.

The description is closed by mentioning some additional advantages of composite light adjustable IOLs 100. (1) Composite light adjustable IOLs 100 can reduce the volume of the photo-polymerizable materials relative to the silicone-only LALs, as the acrylic IOL 110, or acrylic intraocular insert 110', can provide a baseline optical power of 10D, 15D or even 20D. Thus, the silicone-based LAL 120 can be a much thinner layer that is used only for providing the change of the optical power relative to the baseline optical power of the acrylic IOL 110/insert 110'. The smaller volume of the photopolymerizable material translates to smaller intensity and radiance of the refraction modulating illumination, thereby further increasing the safety of the procedure.

(2) In some cases, the refraction modulating illumination has competing effects on the two surfaces of the silicone-based LAL 120. As illustrated in **FIG. 14B,** in some cases the radius of curvature of the proximal surface of the silicone-based LAL 120 can decrease, thereby increasing its optical power. However, the same illumination can increase the radius of curvature of the distal surface, decreasing the optical power of the silicone-based LAL 120. These two effects compete, thus fractionally reducing the efficiency of the refraction modulating illumination. Embodiments of the CLA IOL 100 attach the distal surface of the silicone-based LAL 120 to the IOL 110, or to the acrylic intraocular insert 110'. This attachment increases the rigidity and resistance of the distal surface of the silicone-based LAL 120 against curvature changes, thereby reducing the competition against the optical power increase induced by the proximal surface of the silicone-based LAL 120. Reducing the distal surface curvature change is one more beneficial effect of CLA IOLs 100, as it reduces the radiance of the illumination necessary to achieve a planned optical power change, thereby further increasing retinal safety.

## Claims

**1.** A composite light adjustable intraocular lens, comprising:

an acrylic intraocular insert;
a silicone-based light adjustable lens, attached to the acrylic intraocular insert with an adhesion promoter, wherein the silicone-based light adjustable lens comprises a first polymer matrix and a refraction modulating composition dispersed in the first polymer matrix, wherein the refraction modulating composition is capable of stimulus-induced polymerization that modulates a refraction of the light adjustable lens; and
haptics; wherein
the adhesion promoter includes

a first orthogonal functional group, configured to form a covalent chemical bond with an acrylic component of the acrylic intraocular insert; and
a second orthogonal functional group, configured to form a covalent chemical bond with a silicone component of the silicone-based light adjustable lens.

**2.** The composite light adjustable intraocular lens of claim 1, **characterized by** at least one of:

the haptics being attached to the acrylic intraocular insert;
the haptics being part of the acrylic intraocular insert;
the haptics being attached to the light adjustable lens; and
the haptics being attached to both the acrylic intraocular insert and the light adjustable lens.

**3.** The composite light adjustable intraocular lens of claim 1, wherein:
the acrylic intraocular insert includes a carrier with approximately zero optical power.

**4.** The composite light adjustable intraocular lens of claim 1, wherein:
the acrylic intraocular insert includes an intraocular lens with an optical power.

**5.** The composite light adjustable intraocular lens of claim 1, wherein:
the silicone-based light adjustable lens is attached to the acrylic intraocular insert proximally relative to light incident from the pupil of the eye.

**6.** The composite light adjustable intraocular lens of claim 1, the acrylic intraocular insert comprising:

at least one of a monomer, a macromer, an oligomer and a polymer, selected from the group consisting of an acrylate, an alkyl acrylate, an aryl acrylate, a substituted alkyl acrylate, a substituted aryl acrylate, a halogen substituted acrylate or methacrylate, a halogen substituted aryl acrylate or methacrylate, an acrylic ester, an acrylic acid, a vinyl, and a copolymer combining alkyl acrylates and aryl acrylates,
optionally wherein the monomer is selected from the group consisting of:
a methyl acrylate, an ethyl acrylate, an ethyl hexyl acrylate, a phenyl acrylate, an ethyl methacrylate, a trifluoro ethyl acrylate, a trifluoro ethyl methacrylate, an n-butyl acrylate, a hydroxy ethyl acrylate, a hydroxy methyl acrylate, an n-vinyl pyrrolidone, a phenoxyethyl acrylate, or polymers or co-polymers thereof.

**7.** The composite light adjustable intraocular lens of claim 1, wherein:

(i) the first polymer matrix comprises:
a siloxane-based polymer, formed from macromer and monomer building blocks with at least one of an alkyl group and an aryl group; or
(ii) the light adjustable lens comprises:

a photoinitiator, configured

to be activated upon absorbing a refraction modulating illumination; and
to initiate the stimulus-induced polymerization of the refraction modulating compound; and

an ultraviolet absorber.

**8.** The composite light adjustable intraocular lens of claim 1, wherein:
the adhesion promoter is dispersed in the acrylic intraocular insert.

**9.** The composite light adjustable intraocular lens of claim 1, wherein:
the adhesion promoter is dispersed in an adhesion layer between the acrylic intraocular insert and the light adjustable lens.

**10.** The composite light adjustable intraocular lens of claim 1, wherein:
the adhesion promoter is dispersed in the light adjustable lens.

**11.** The composite light adjustable intraocular lens of claim 1, wherein:

the adhesion promoter has the structure

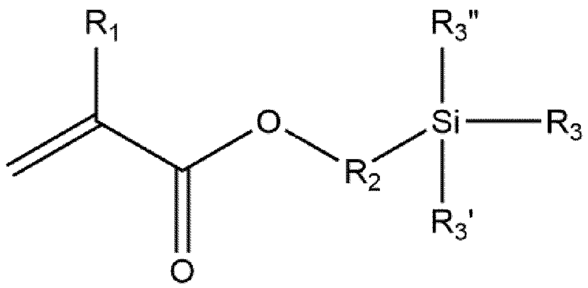

;

wherein

at least one of $R_3$, $R_3$' and $R_3$" is a vinyl dialkylsiloxy pendant group with the structure

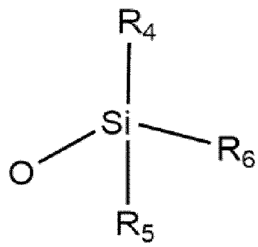

;

the remaining of $R_3$, $R_3$' and $R_3$" are independently selected from the group consisting of C1-C10 pendant alkyl groups, such as methyl, ethyl, n-propyl, isopropyl, cyclopropyl, n-butyl, sec-butyl, t-butyl, cyclobutyl, or methylcyclopropyl;

the first orthogonal functional group is the functional group to the left of $R_2$;

the second orthogonal functional group is $R_6$;

$R_1$ is selected from the group consisting of a hydrogen, a monovalent hydrocarbon group, and a substituted C1-C12 alkyl, wherein the alkyl can be methyl, ethyl, n-propyl, isopropyl, cyclopropyl, n-butyl, sec-butyl, t-butyl, cyclobutyl, or methylcyclopropyl;

$R_2$ is an alkyl spacer with 1-10 carbon atoms, such as $(-CH_2)_n$, where n=1 through 10;

$R_4$ and $R_5$ are independently selected from the group consisting of C1-C10 pendant alkyl groups, such as methyl, ethyl, n-propyl, isopropyl, cyclopropyl, n-butyl, sec-butyl, t-butyl, cyclobutyl, or methylcyclopropyl; and

$R_6$ is one of a vinyl group, a vinyloxy group, an allyl group, an allyloxy group, and a group with a carbon chain C1-C10, optionally wherein:

(i) $R_1$ is methyl, $R_2$ is propyl, $R_3$, $R_3$' and $R_3$" each is vinyl dialkylsiloxy, $R_4$ and $R_5$ each is C1-10 alkyl chain, and $R_6$ is vinyl; or

(ii) the adhesion promoter comprises methacryloxypropyltris(vinyldimethylsiloxy)silane with the structure

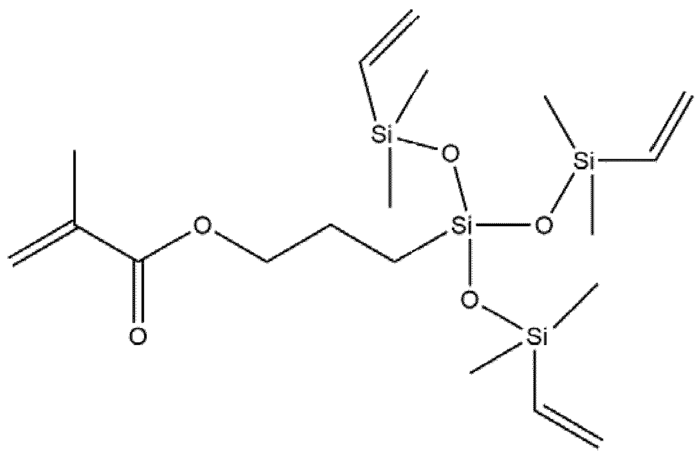

;

or

(iii) the adhesion promoter is dispersed in the acrylic intraocular insert with a concentration greater than 5 weight%.

**12.** The composite light adjustable intraocular lens of claim 11, wherein:
silicon-carbon covalent bonds are created via a hydrosilation reaction between the vinyldialkylsiloxy pendant group on at least one of the pendant groups and an Si-H group of the silicone-based light adjustable lens.

**13.** The composite light adjustable intraocular lens of claim 1, the light adjustable lens comprising:
an ultraviolet absorbing layer at a distal surface of the light adjustable lens, wherein the distal surface is distal relative to light incident from the pupil of the eye.

**14.** The composite light adjustable intraocular lens of claim 1, wherein:

the light adjustable lens is attached to the acrylic intraocular insert at a proximal surface of the acrylic intraocular insert, wherein the proximal surface is proximal relative to light incident from the pupil of the eye; and
the acrylic intraocular insert includes at least one of

an ultraviolet absorbing material dispersed throughout the acrylic intraocular insert; and
an ultraviolet absorbing layer formed at least one of a proximal and a distal surface of the acrylic intraocular insert, wherein the proximal surface is proximal relative to light incident from the pupil of the eye and the distal surface is distal relative to light incident from the pupil of the eye.

**15.** The composite light adjustable intraocular lens of claim 1, wherein:

(i) the light adjustable lens is attached to the acrylic intraocular insert by at least one of a chemical reaction, a thermal treatment, an illumination treatment, a polymerization process, a molding step, a curing step, a lathing step, a cryo-lathing step, a mechanical process, an application of an adhesive, and by a combination thereof; or
(ii) the acrylic intraocular insert is a diffractive intraocular lens with an optical power; or
(iii) the acrylic intraocular insert is a toric acrylic intraocular insert.

**Patentansprüche**

**1.** Zusammengesetzte lichtanpassbare Intraokularlinse, die umfasst:

einen Intraokulareinsatz aus Acryl;
eine lichtanpassbare Linse auf Silikonbasis, die mit einem Haftvermittler an dem Intraokulareinsatz aus Acryl befestigt ist, wobei die lichtanpassbare Linse auf Silikonbasis eine erste Polymermatrix und eine in der ersten Polymermatrix dispergierte refraktionsmodulierende Zusammensetzung umfasst, wobei die refraktionsmodulierende Zusammensetzung zu einer durch Stimuli induzierten Polymerisation fähig ist, die eine Refraktion der lichtanpassbaren Linse moduliert; und
Haptik; wobei
der Haftvermittler beinhaltet:

eine erste orthogonale funktionelle Gruppe, die dazu konfiguriert ist, eine kovalente chemische Bindung mit einer Komponente aus Acryl des Intraokulareinsatzes aus Acryl auszubilden, und
eine zweite orthogonale funktionelle Gruppe, die dazu konfiguriert ist, eine kovalente chemische Bindung mit einer Silikonkomponente der lichtanpassbaren Linse auf Silikonbasis auszubilden.

**2.** Zusammengesetzte lichtanpassbare Intraokularlinse nach Anspruch 1, **gekennzeichnet durch** wenigstens eines von:

die Haptik ist an dem Intraokulareinsatz aus Acryl befestigt;
die Haptik ist Teil des Intraokulareinsatzes aus Acryl;
die Haptik ist an der lichtanpassbaren Linse befestigt; und
die Haptik ist sowohl an dem Intraokulareinsatz aus Acryl als auch an der lichtanpassbaren Linse befestigt.

**3.** Zusammengesetzte lichtanpassbare Intraokularlinse nach Anspruch 1, wobei:

der Intraokulareinsatz aus Acryl einen Träger mit einer optischen Leistung von ungefähr Null beinhaltet.

**4.** Zusammengesetzte lichtanpassbare Intraokularlinse nach Anspruch 1, wobei:
der Intraokulareinsatz aus Acryl eine Intraokularlinse mit einer optischen Leistung beinhaltet.

**5.** Zusammengesetzte lichtanpassbare Intraokularlinse nach Anspruch 1, wobei:
die lichtanpassbare Linse auf Silikonbasis proximal relativ zu dem Lichteinfall aus der Pupille des Auges befestigt ist.

**6.** Zusammengesetzte lichtanpassbare Intraokularlinse nach Anspruch 1, wobei der Intraokulareinsatz aus Acryl umfasst:

wenigstens eines von einem Monomer, einem Makromer, einem Oligomer und einem Polymer, ausgewählt aus der Gruppe, bestehend aus einem Acrylat, einem Alkylacrylat, einem Arylacrylat, einem substituierten Alkylacrylat, einem substituierten Arylacrylat, einem halogensubstituierten Acrylat oder Methacrylat, einem halogensubstituierten Arylacrylat oder Methacrylat, einem Acrylester, einer Acrylsäure, einem Vinyl und einem Copolymer, der Alkylacrylate und Arylacrylate kombiniert,
wobei das Monomer optional aus der Gruppe ausgewählt ist, bestehend aus:
einem Methylacrylat, einem Ethylacrylat, einem Ethylhexylacrylat, einem Phenylacrylat, einem Ethylmethacrylat, einem Trifluorethylacrylat, einem Trifluorethylmethacrylat, einem n-Butylacrylat, einem Hydroxyethylacrylat, einem Hydroxymethylacrylat, einem n-Vinylpyrrolidon, einem Phenoxyethylacrylat oder Polymeren oder Copolymeren davon.

**7.** Zusammengesetzte lichtanpassbare Intraokularlinse nach Anspruch 1, wobei:

(i) die erste Polymermatrix umfasst:
ein Polymer auf Siloxanbasis, ausgebildet aus Makromer- und Monomerbausteinen mit wenigstens einem von einer Alkylgruppe und einer Arylgruppe; oder
(ii) die lichtanpassbare Linse umfasst:

einen Photoinitiator, der dazu konfiguriert ist,

bei Absorption einer die Brechung modulierenden Beleuchtung aktiviert zu werden; und
die durch Stimuli induzierte Polymerisation der die Brechung modulierenden Verbindung zu initiieren; und

einen Ultraviolettabsorber.

**8.** Zusammengesetzte lichtanpassbare Intraokularlinse nach Anspruch 1, wobei:
der Haftvermittler in dem Intraokulareinsatz aus Acryl dispergiert ist.

**9.** Zusammengesetzte lichtanpassbare Intraokularlinse nach Anspruch 1, wobei:
der Haftvermittler in einer Haftschicht zwischen dem Intraokulareinsatz aus Acryl und der lichtanpassbaren Linse dispergiert ist.

**10.** Zusammengesetzte lichtanpassbare Intraokularlinse nach Anspruch 1, wobei:
der Haftvermittler in der lichtanpassbaren Linse dispergiert ist.

**11.** Zusammengesetzte lichtanpassbare Intraokularlinse nach Anspruch 1, wobei:

der Haftvermittler die Struktur aufweist:

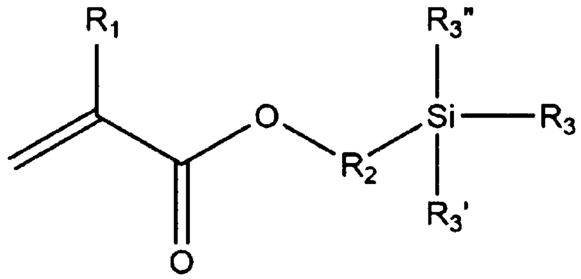

;

wobei

wenigstens eines von $R_3$, $R_3$' und $R_3$" eine vinyldialkylsiloxyüberhängende Gruppe mit der Struktur ist:

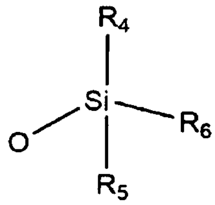

;

die übrigen $R_3$, $R_3$' und $R_3$'' unabhängig aus der Gruppe ausgewählt sind, bestehend aus C1-C10-überhängenden Alkylgruppen wie Methyl, Ethyl, n-Propyl, Isopropyl, Cyclopropyl, n-Butyl, sec-Butyl, t-Butyl, Cyclobutyl oder Methylcyclopropyl;

die erste orthogonale funktionelle Gruppe die funktionelle Gruppe links von $R_2$ ist;

die zweite orthogonale funktionelle Gruppe $R_6$ ist;

$R_1$ aus der Gruppe ausgewählt ist, die aus Wasserstoff, einer einwertigen Kohlenwasserstoffgruppe und einem substituierten C 1-C12-Alkyl besteht, wobei das Alkyl Methyl, Ethyl, n-Propyl, Isopropyl, Cyclopropyl, n-Butyl, sec-Butyl, t-Butyl, Cyclobutyl oder Methylcyclopropyl sein kann;

$R_2$ ein Alkyl-Spacer mit 1-10 Kohlenstoffatomen ist, wie $(-CH_2)_n$, wobei n = 1 bis einschließlich 10 ist;

$R_4$ und $R_5$ unabhängig aus der Gruppe ausgewählt sind, bestehend aus C1-C10-überhängenden Alkylgruppen, wie Methyl, Ethyl, n-Propyl, Isopropyl, Cyclopropyl, n-Butyl, sec-Butyl, t-Butyl, Cyclobutyl oder Methylcyclopropyl; und

$R_6$ eines von einer Vinylgruppe, einer Vinyloxygruppe, einer Allylgruppe, einer Allyloxygruppe und einer Gruppe mit einer Kohlenstoffkette C1-C10 ist, wobei optional:

(i) $R_1$ Methyl ist, $R_2$ Propyl ist, $R_3$, $R_3$' und $R_3$" jeweils Vinyldialkylsiloxy sind, $R_4$ und $R_5$ jeweils eine C1-10-Alkylkette sind und $R_6$ Vinyl ist; oder

(ii) der Haftvermittler Methacryloxypropyltris(vinyldimethylsiloxy)silan mit der Struktur umfasst:

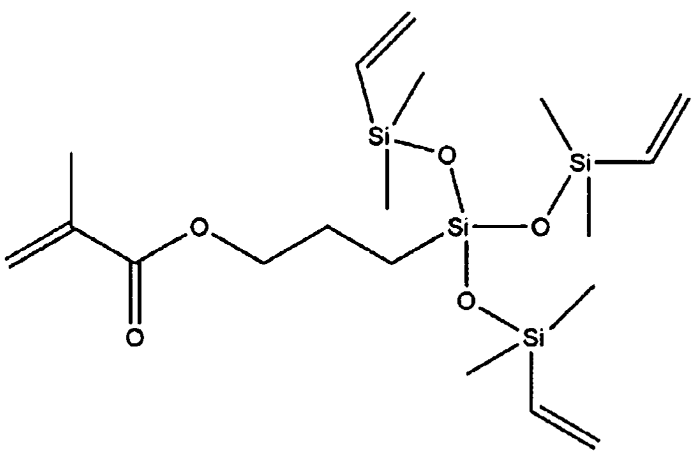

;

oder

(iii) der Haftvermittler in dem Intraokulareinsatz aus Acryl in einer Konzentration von mehr als 5 Gew.-% dispergiert ist.

**12.** Zusammengesetzte lichtanpassbare Intraokularlinse nach Anspruch 11, wobei:
kovalente Silizium-Kohlenstoff-Bindungen durch eine Hydrosilylierungsreaktion zwischen der vinyldialkylsiloxyüberhängenden Gruppe an wenigstens einer der überhängenden Gruppen und einer Si-H-Gruppe der lichtanpassbaren Linse auf Silikonbasis gebildet werden.

**13.** Zusammengesetzte lichtanpassbare Intraokularlinse nach Anspruch 1, wobei die lichtanpassbare Linse umfasst:
eine ultraviolettabsorbierende Schicht an einer distalen Oberfläche der lichtanpassbaren Linse, wobei die distale Oberfläche distal relativ zu dem von der Pupille des Auges einfallenden Licht ist.

**14.** Zusammengesetzte lichtanpassbare Intraokularlinse nach Anspruch 1, wobei:

die lichtanpassbare Linse an der proximalen Oberfläche des Intraokulareinsatzes aus Acryl befestigt ist, wobei die proximale Oberfläche proximal relativ zu dem von der Pupille des Auges einfallenden Licht ist; und
der Intraokulareinsatz aus Acryl wenigstens eines beinhaltet von:

einem ultraviolettabsorbierenden Material, das in dem gesamten Intraokulareinsatz aus Acryl dispergiert ist; und
einer ultraviolettabsorbierenden Schicht, die wenigstens an einem von einer proximalen und einer distalen Oberfläche des Intraokulareinsatzes aus Acryl ausgebildet ist, wobei die proximale Oberfläche relativ zu dem von der Pupille des Auges einfallenden Licht proximal ist und die distale Oberfläche relativ zu dem von der Pupille des Auges einfallenden Licht distal ist.

**15.** Zusammengesetzte lichtanpassbare Intraokularlinse nach Anspruch 1, wobei:

(i) die lichtanpassbare Linse durch wenigstens eines von einer chemische Reaktion, einer thermischen Behandlung, einer Bestrahlungsbehandlung, einem Polymerisationsprozess, einem Formungsschritt, einem Aushärtungsschritt, einem Belattungsschritt, einem Kryo-Belattungsschritt, einem mechanischen Prozess, einem Auftragen eines Klebstoffs und einer Kombination davon an dem Intraokulareinsatz aus Acryl befestigt ist; oder
(ii) der Intraokulareinsatz aus Acryl eine diffraktive Intraokularlinse mit einer optischen Leistung ist; oder
(iii) der Intraokulareinsatz aus Acryl ein torischer Intraokulareinsatz aus Acryl ist.

## Revendications

**1.** Lentille intraoculaire composite ajustable à la lumière comprenant :

un insert intraoculaire en acrylique,
une lentille ajustable à la lumière à base de silicone, fixée à l'insert intraoculaire en acrylique avec un promoteur d'adhérence, ladite lentille ajustable à la lumière à base de silicone comprenant une première matrice polymère et une composition modulant la réfraction dispersée dans la première matrice polymère, ladite composition modulant la réfraction étant apte à une polymérisation induite par stimulus qui module la réfraction de lentille ajustable à la lumière, et
des haptiques ;
ledit promoteur d'adhérence comprenant

un premier groupe fonctionnel orthogonal configuré pour former une liaison chimique covalente avec un composant acrylique de l'insert intraoculaire en acrylique, et
un deuxième groupe fonctionnel orthogonal configuré pour former une liaison chimique covalente avec un composant silicone de la lentille ajustable à la lumière à base de silicone.

**2.** Lentille intraoculaire composite ajustable à la lumière selon la revendication 1, **caractérisée par** au moins une des caractéristiques suivantes :

les haptiques sont fixées à l'insert intraoculaire en acrylique ;
les haptiques font partie de l'insert intraoculaire en acrylique ;

les haptiques sont fixées à la lentille ajustable à la lumière ; et
les haptiques sont fixées à la fois à l'insert intraoculaire en acrylique et à la lentille ajustable à la lumière.

**3.** Lentille intraoculaire composite ajustable à la lumière selon la revendication 1, dans laquelle : l'insert intraoculaire en acrylique comprend un support doté d'une puissance optique approximativement nulle.

**4.** Lentille intraoculaire composite ajustable à la lumière selon la revendication 1, dans laquelle : l'insert intraoculaire en acrylique comprend une lentille intraoculaire dotée d'une puissance optique.

**5.** Lentille intraoculaire composite ajustable à la lumière selon la revendication 1, dans laquelle : la lentille ajustable à la lumière à base de silicone est fixée à l'insert intraoculaire en acrylique proximalement par rapport à la lumière incidente de la pupille de l'œil.

**6.** Lentille intraoculaire composite ajustable à la lumière selon la revendication 1, l'insert intraoculaire en acrylique comprenant :

au moins un composant parmi un monomère, macromère, oligomère et polymère, choisi dans le groupe constitué par un acrylate, acrylate d'alkyle, acrylate d'aryle, acrylate d'alkyle substitué, acrylate d'aryle substitué, acrylate ou méthacrylate substitué par un halogène, acrylate ou méthacrylate d'aryle substitué par un halogène, ester acrylique, acide acrylique, vinyle, et un copolymère combinant des acrylates d'alkyle et acrylates d'aryle,
ledit monomère étant éventuellement choisi dans le groupe constitué par :
acrylate de méthyle, acrylate d'éthyle, acrylate d'éthylhexyle, acrylate de phényle, méthacrylate d'éthyle, acrylate de trifluoroéthyle, méthacrylate de trifluoroéthyle, acrylate de n-butyle, acrylate d'hydroxyéthyle, acrylate d'hydroxyméthyle, n-vinylpyrrolidone, acrylate de phénoxyéthyle, ou polymères ou copolymères de ceux-ci.

**7.** Lentille intraoculaire composite ajustable à la lumière selon la revendication 1, dans laquelle :

(i) la première matrice polymère comprend :
un polymère à base de siloxane formé à partir de blocs de construction macromères et monomères avec un groupe alkyle et/ou un groupe aryle ; ou
(ii) la lentille ajustable à la lumière comprend :

un photoamorceur configuré pour

être activé lors de l'absorption d'une illumination modulant la réfraction, et
amorcer la polymérisation induite par stimulus du composé modulant la réfraction ; et

un absorbeur d'ultraviolets.

**8.** Lentille intraoculaire composite ajustable à la lumière selon la revendication 1, dans laquelle : le promoteur d'adhérence est dispersé dans l'insert intraoculaire en acrylique.

**9.** Lentille intraoculaire composite ajustable à la lumière selon la revendication 1, dans laquelle : le promoteur d'adhérence est dispersé dans une couche d'adhérence entre l'insert intraoculaire en acrylique et la lentille ajustable à la lumière.

**10.** Lentille intraoculaire composite ajustable à la lumière selon la revendication 1, dans laquelle : le promoteur d'adhérence est dispersé dans la lentille ajustable à la lumière.

**11.** Lentille intraoculaire composite ajustable à la lumière selon la revendication 1, dans laquelle :

le promoteur d'adhérence présente la structure

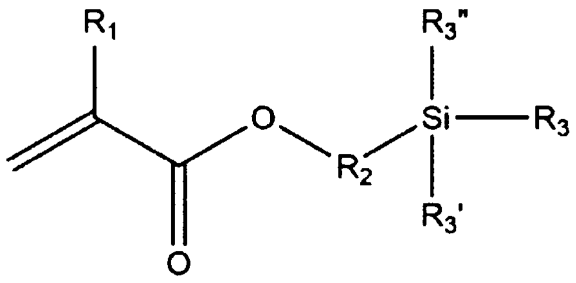

;

où

au moins un groupe parmi $R_3$, $R_3$' et $R_3$" est un groupe pendant vinyl dialkylsiloxy présentant la structure

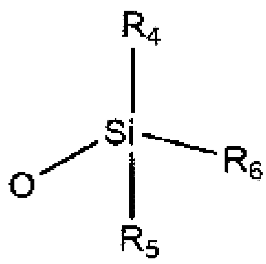

;

les autres groupes parmi $R_3$, $R_3$' et $R_3$" sont choisis indépendamment dans le groupe constitué par les groupes alkyles pendants en C1-C10, tels que méthyle, éthyle, n-propyle, isopropyle, cyclopropyle, n-butyle, sec-butyle, t-butyle, cyclobutyle ou méthylcyclopropyle ;

le premier groupe fonctionnel orthogonal est le groupe fonctionnel situé à gauche de $R_2$ ;

le deuxième groupe fonctionnel orthogonal est $R_6$ ;

$R_1$ est choisi dans le groupe constitué par un hydrogène, un groupe hydrocarboné monovalent et un alkyle en C1-C12 substitué, ledit alkyle pouvant être méthyle, éthyle, n-propyle, isopropyle, cyclopropyle, n-butyle, sec-butyle, t-butyle, cyclobutyle ou méthylcyclopropyle ;

$R_2$ est un espaceur alkyle avec 1 à 10 atomes de carbone, tel que $(-CH_2)_n$, où n = 1 à 10 ;

$R_4$ et $R_5$ sont choisis indépendamment dans le groupe constitué par les groupes alkyles pendants en C1-C10, tels que méthyle, éthyle, n-propyle, isopropyle, cyclopropyle, n-butyle, sec-butyle, t-butyle, cyclobutyle ou méthylcyclopropyle ; et

$R_6$ est un groupe parmi : un groupe vinyle, un groupe vinyloxy, un groupe allyle, un groupe allyloxy et un groupe à chaîne carbonée en C1-C10, étant entendu éventuellement que :

(i) $R_1$ représente méthyle, $R_2$ représente propyle, $R_3$, $R_3$' et $R_3$" représentent chacun vinyle dialkylsiloxy, $R_4$ et $R_5$ représentent chacun une chaîne alkyle en C1-C10 et $R_6$ représente vinyle ; ou

(ii) le promoteur d'adhérence comprend du méthacryloxypropyltris(vinyldimenthylsiloxy)silane présentant la structure

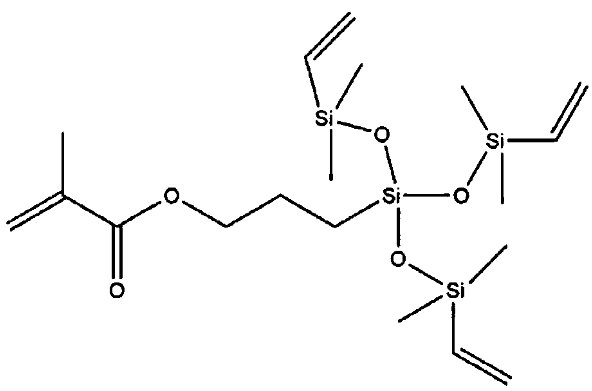

;

ou

(iii) le promoteur d'adhérence est dispersé dans l'insert intraoculaire en acrylique avec une concentration supérieure à 5 % en poids.

**12.** Lentille intraoculaire composite ajustable à la lumière selon la revendication 11, dans laquelle :
des liaisons covalentes silicium-carbone sont créées par une réaction d'hydrosilylation entre le groupe pendant vinyldialkylsiloxy sur au moins l'un des groupes pendants et un groupe Si-H de la lentille ajustable à la lumière à base de silicone.

**13.** Lentille intraoculaire composite ajustable à la lumière selon la revendication 1, la lentille ajustable à la lumière comprenant :
une couche absorbant les ultraviolets sur une surface distale de la lentille ajustable à la lumière, ladite surface distale étant distale par rapport à la lumière incidente de la pupille de l'œil.

**14.** Lentille intraoculaire composite ajustable à la lumière selon la revendication 1, dans laquelle :

la lentille ajustable à la lumière est fixée à l'insert intraoculaire en acrylique sur une surface proximale de l'insert intraoculaire en acrylique, ladite surface proximale étant proximale par rapport à la lumière incidente de la pupille de l'œil ; et
l'insert intraoculaire en acrylique comprend au moins un constituant parmi :

un matériau absorbant les ultraviolets dispersé dans tout l'insert intraoculaire en acrylique, et
une couche absorbant les ultraviolets formée sur une surface proximale et/ou une surface distale de l'insert intraoculaire en acrylique, ladite surface proximale étant proximale par rapport à la lumière incidente de la pupille de l'œil et la surface distale étant distale par rapport à la lumière incidente de la pupille de l'œil.

**15.** Lentille intraoculaire composite ajustable à la lumière selon la revendication 1, dans laquelle :

(i) la lentille ajustable à la lumière est fixée à l'insert intraoculaire en acrylique par une réaction chimique, un traitement thermique, un traitement par illumination, un processus de polymérisation, une étape de moulage, une étape de durcissement, une étape de tournage, une étape de cryo-tournage, un processus mécanique, une application d'adhésif ou une combinaison de ceux-ci ; ou
(ii) l'insert intraoculaire en acrylique est une lentille intraoculaire diffractive dotée d'une puissance optique ; ou
(iii) l'insert intraoculaire en acrylique est un insert intraoculaire en acrylique torique.

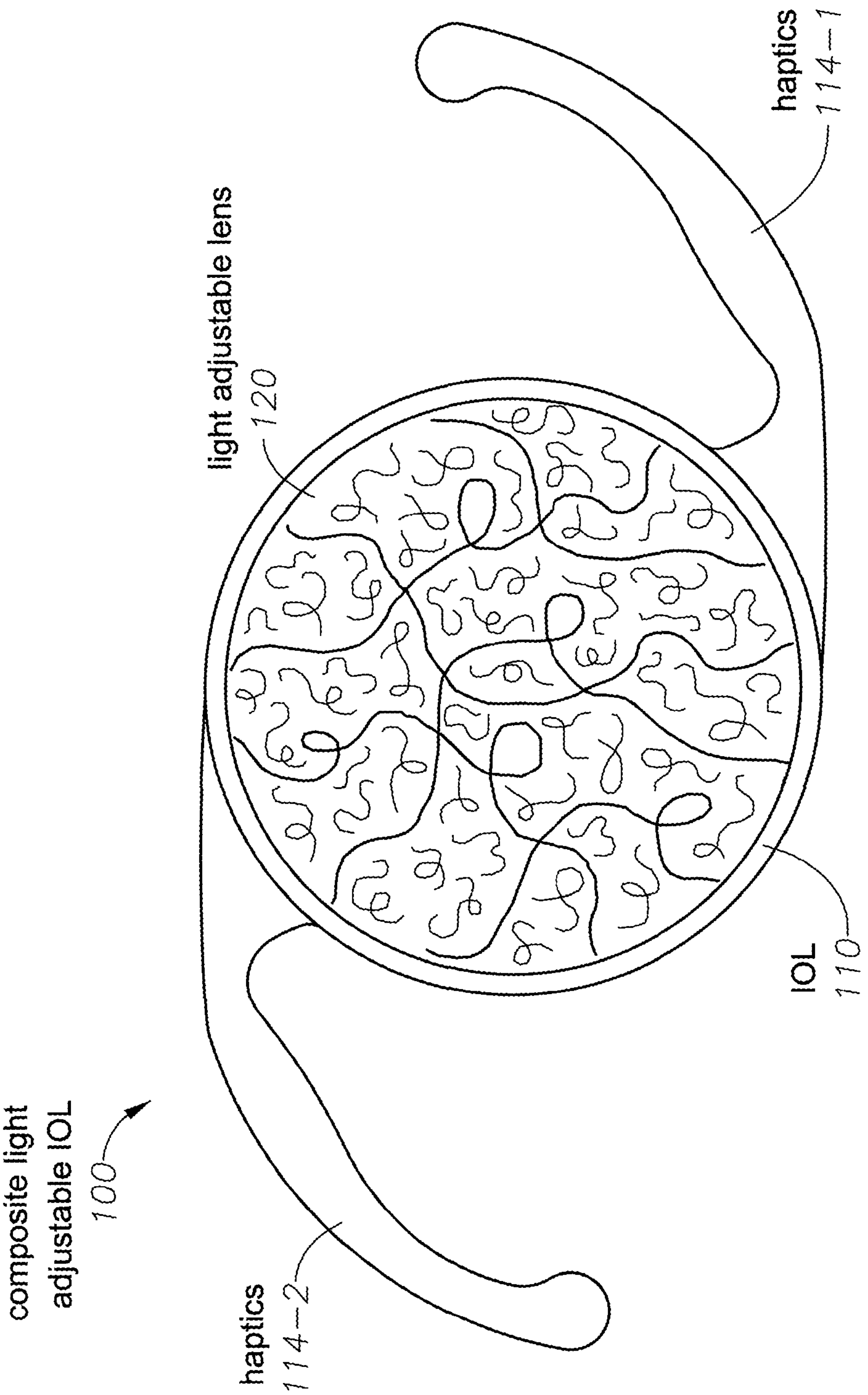
composite light adjustable IOL
100
light adjustable lens
120
haptics
114—1
haptics
114—2
IOL
110

FIG. 1

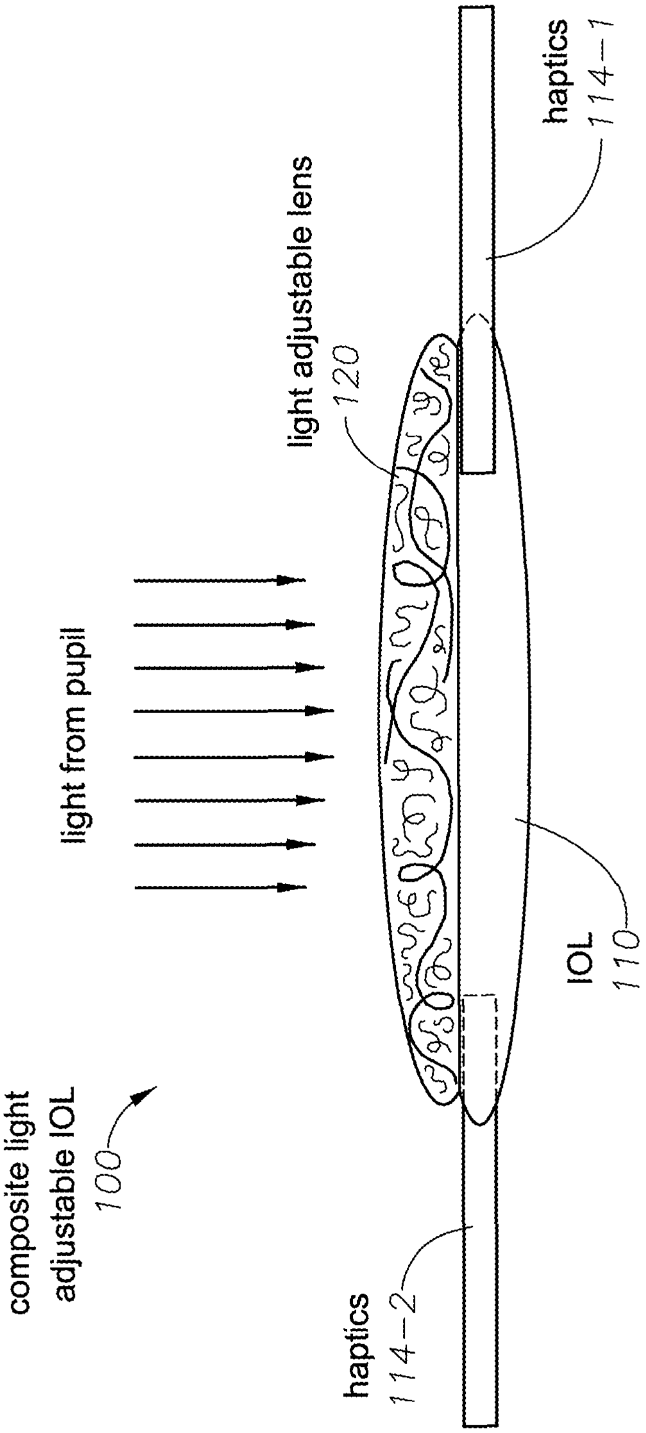
haptics
114-1
light adjustable lens
120
light from pupil
IOL
110
composite light
adjustable IOL
100
haptics
114-2

FIG. 2A

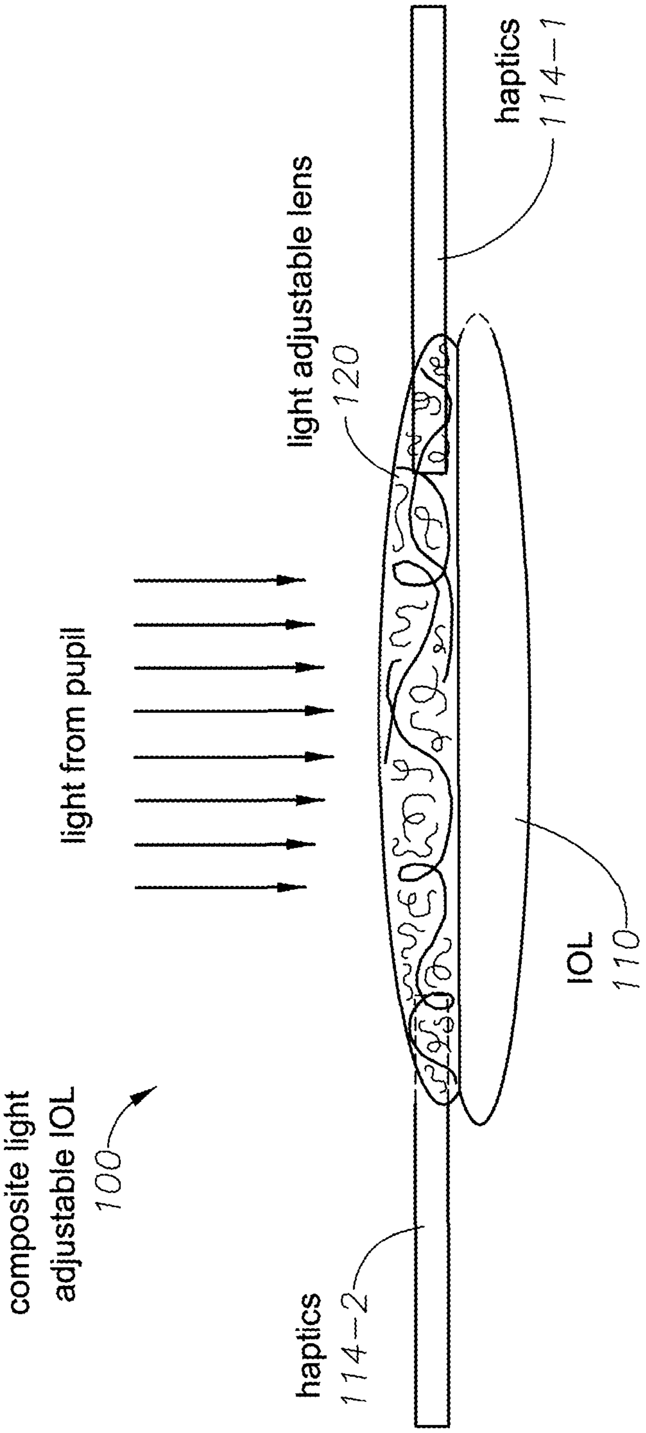
composite light adjustable IOL
100
haptics
114—2
light from pupil
light adjustable lens
120
haptics
114—1
IOL
110

FIG. 2B

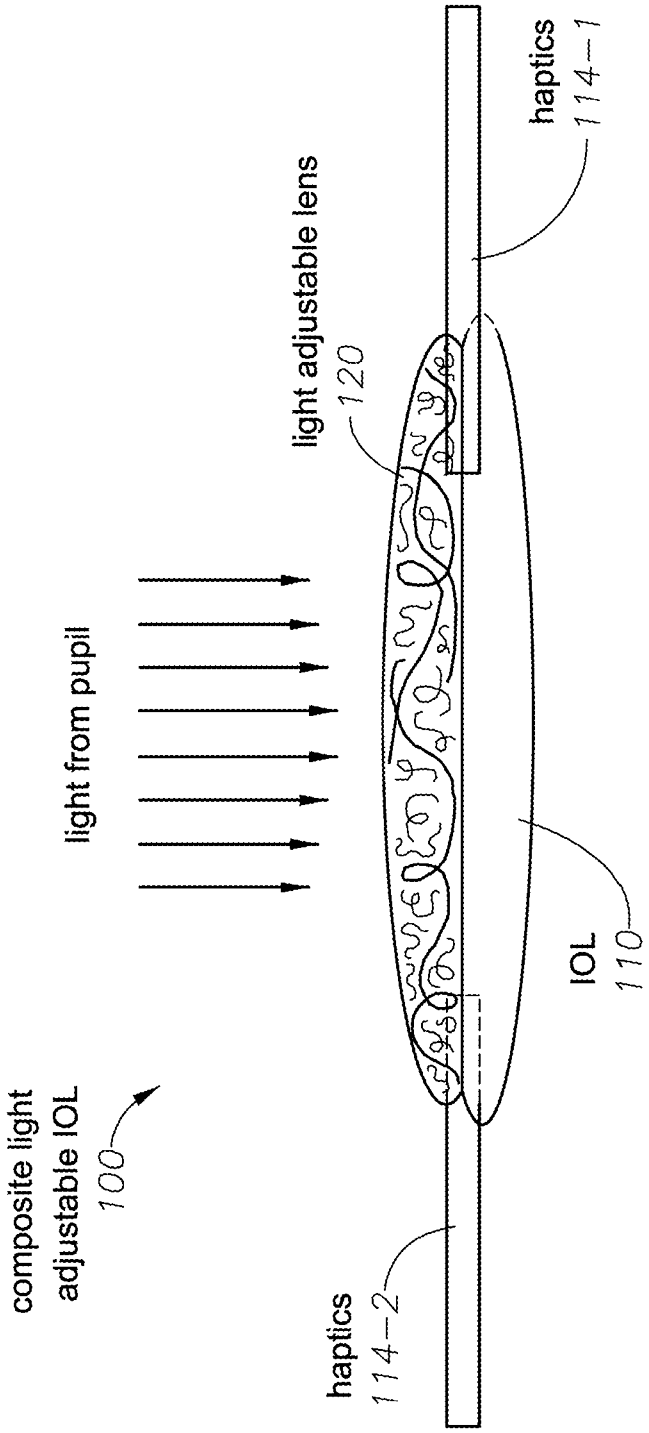
haptics
114-1
light adjustable lens
120
light from pupil
IOL
110
composite light
adjustable IOL
100
haptics
114-2

FIG. 2C

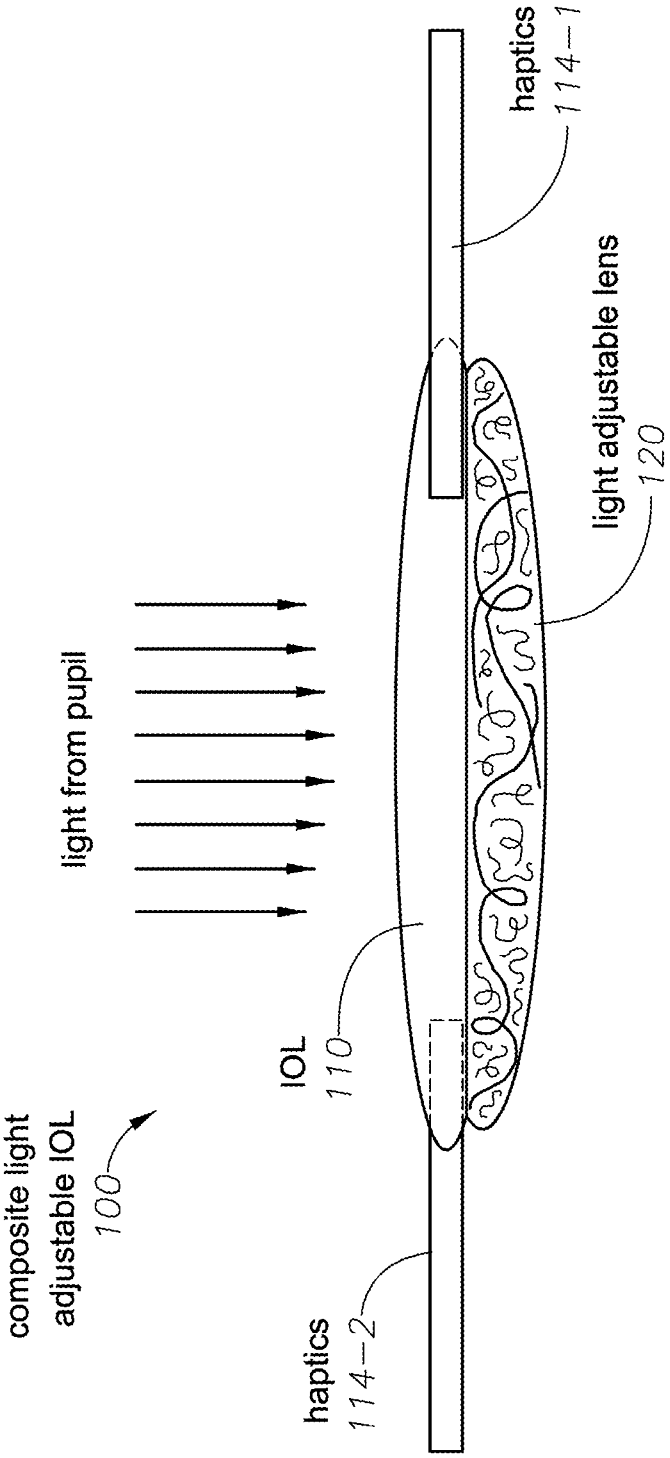
haptics
114-1
light adjustable lens
120
light from pupil
IOL
110
composite light
adjustable IOL
100
haptics
114-2

FIG. 3

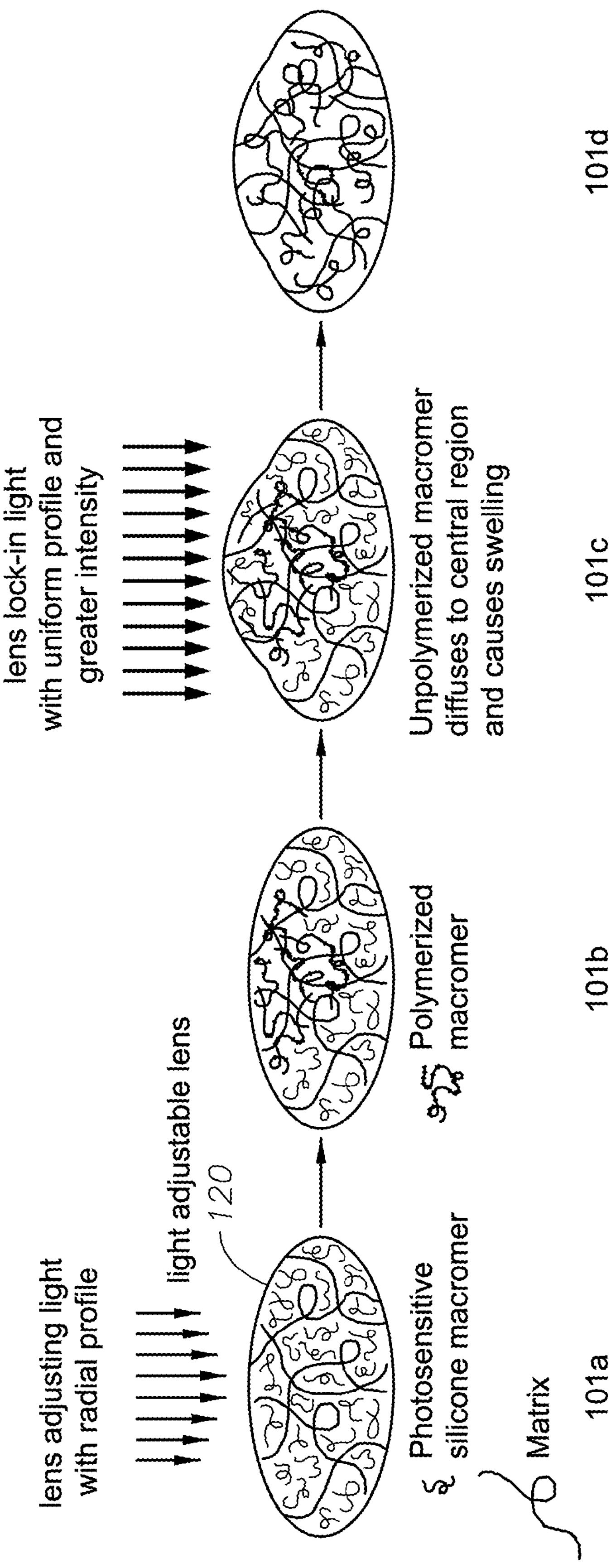
lens adjusting light
with radial profile
light adjustable lens
120
Photosensitive
silicone macromer
Matrix
101a
Polymerized
macromer
101b
lens lock-in light
with uniform profile and
greater intensity
Unpolymerized macromer
diffuses to central region
and causes swelling
101c
101d

FIG. 4

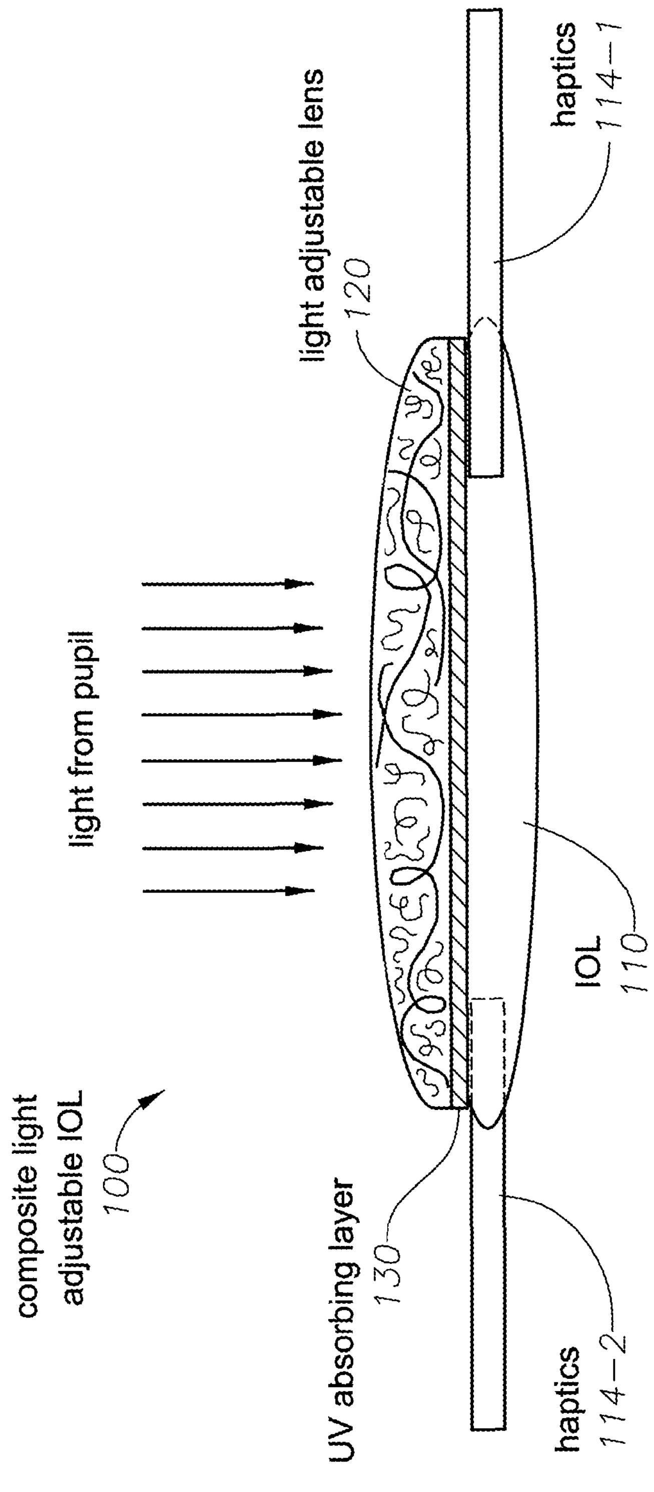
haptics
114-1
light adjustable lens
120
light from pupil
IOL
110
composite light
adjustable IOL
100
UV absorbing layer
130
haptics
114-2

FIG. 5

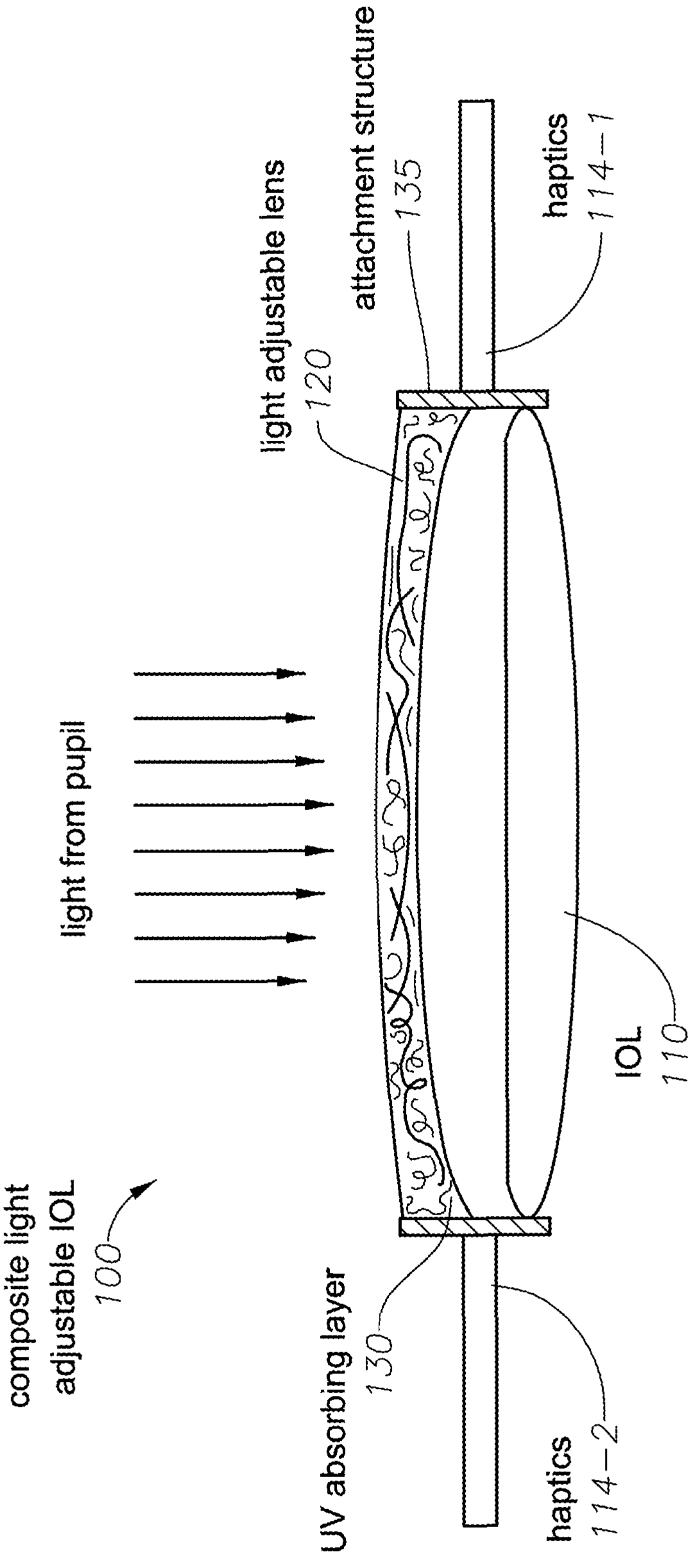
light adjustable lens
120
attachment structure
135
haptics
114-1
light from pupil
IOL
110
composite light
adjustable IOL
100
UV absorbing layer
130
haptics
114-2

FIG. 6

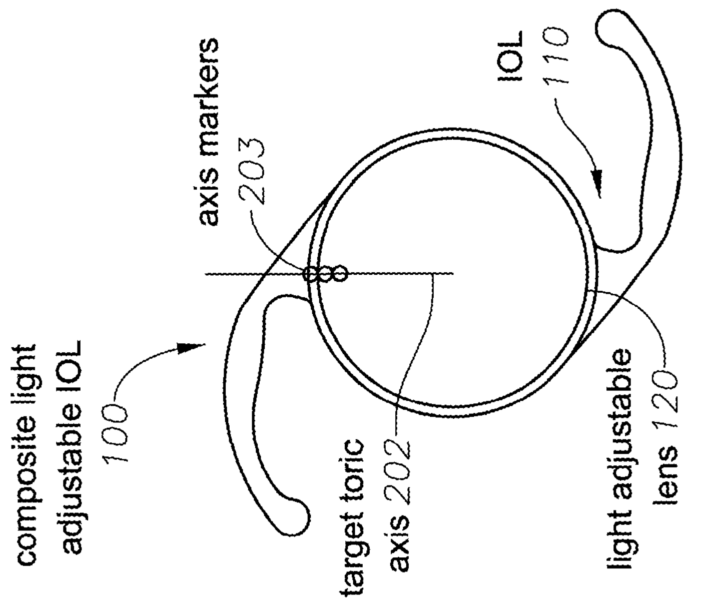


FIG. 7A

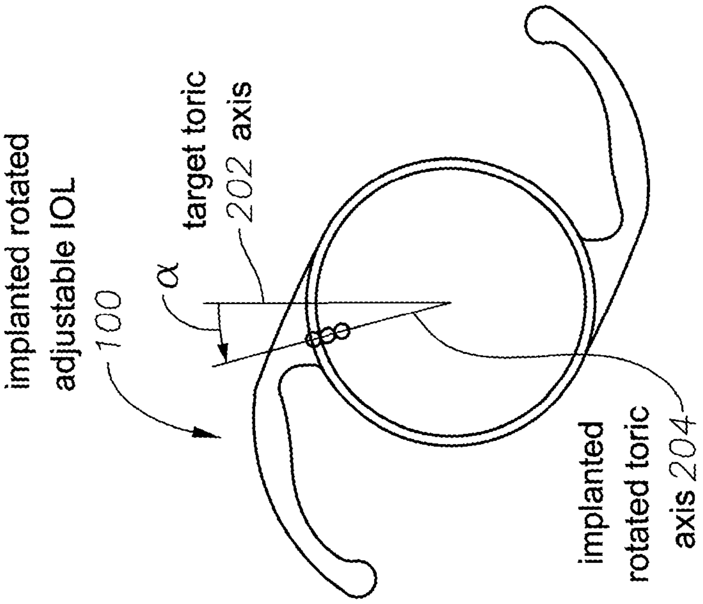


FIG. 7B

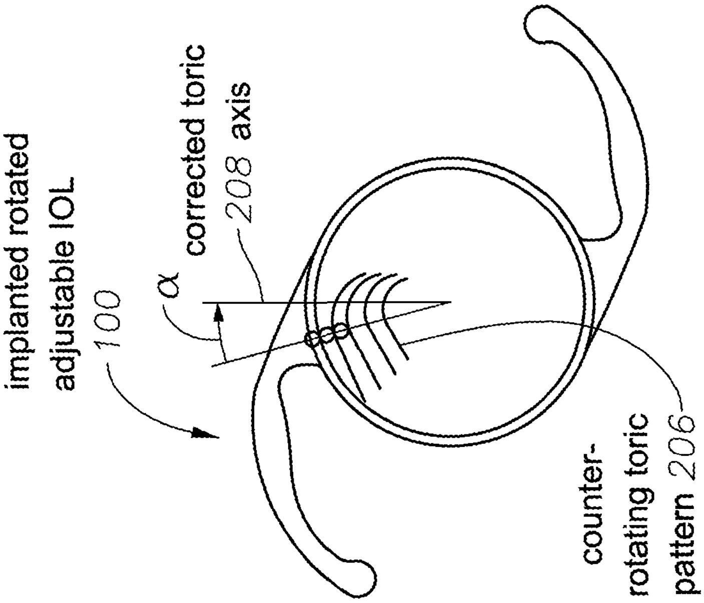


FIG. 7C

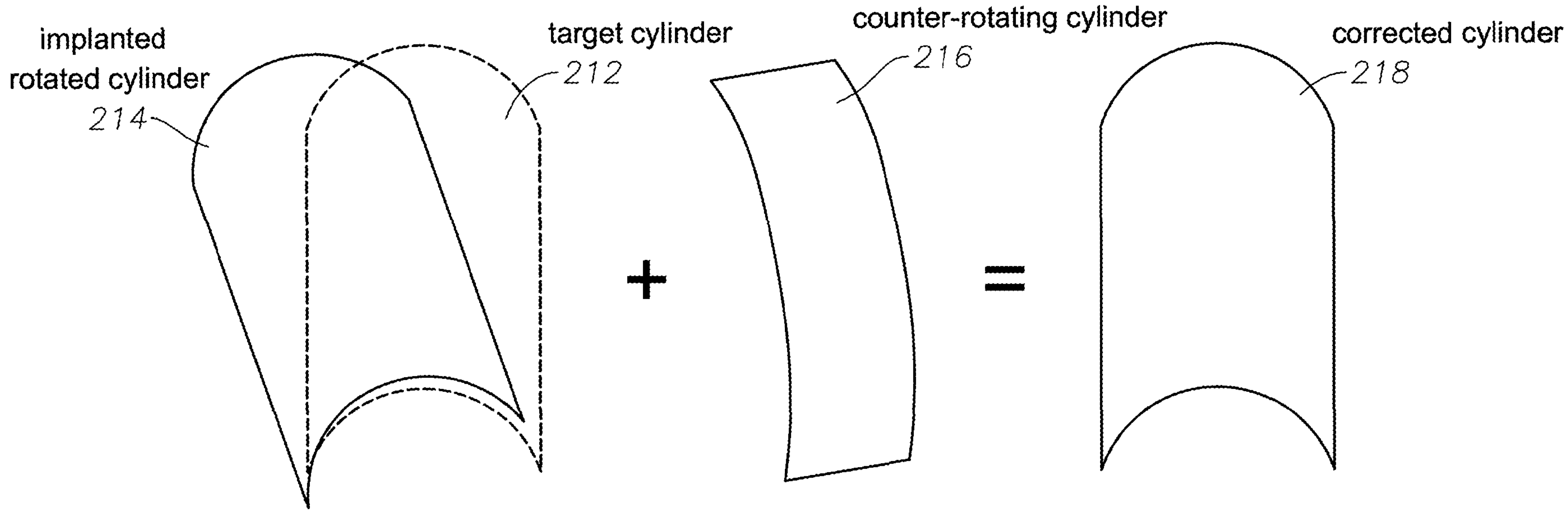
implanted
rotated cylinder
214
target cylinder
212
+
counter-rotating cylinder
216
=
corrected cylinder
218

FIG. 8A

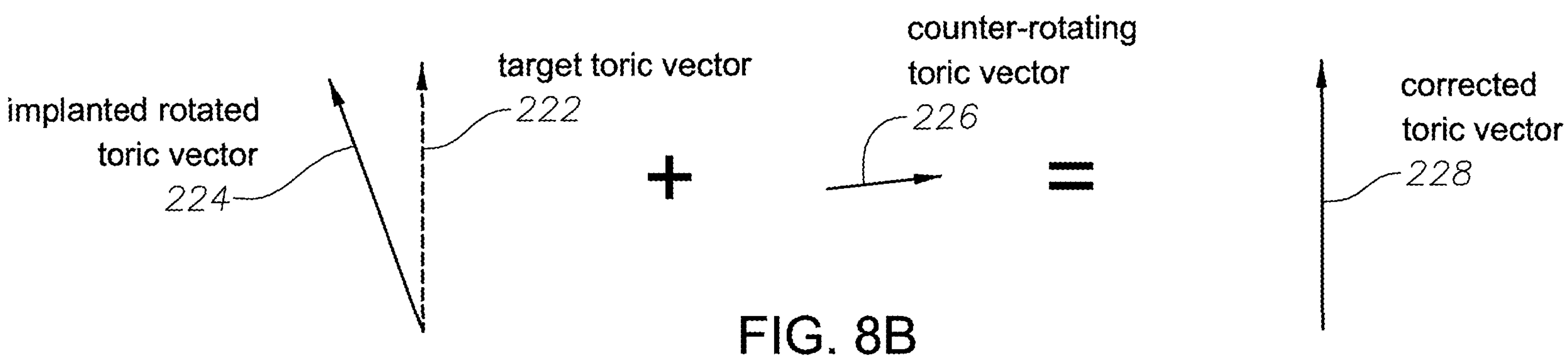
implanted rotated
toric vector
224
target toric vector
222
+
counter-rotating
toric vector
226
=
corrected
toric vector
228

FIG. 8B

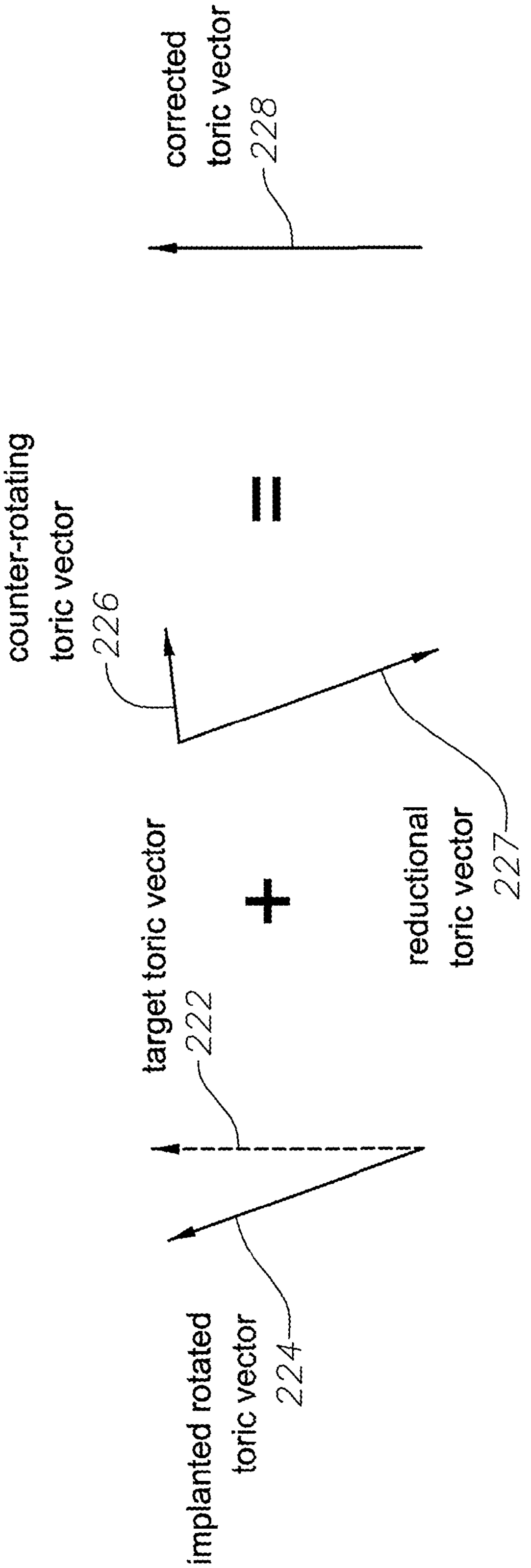
implanted rotated toric vector
224
target toric vector
222
+
reductional toric vector
227
counter-rotating toric vector
226
=
corrected toric vector
228

FIG. 8C

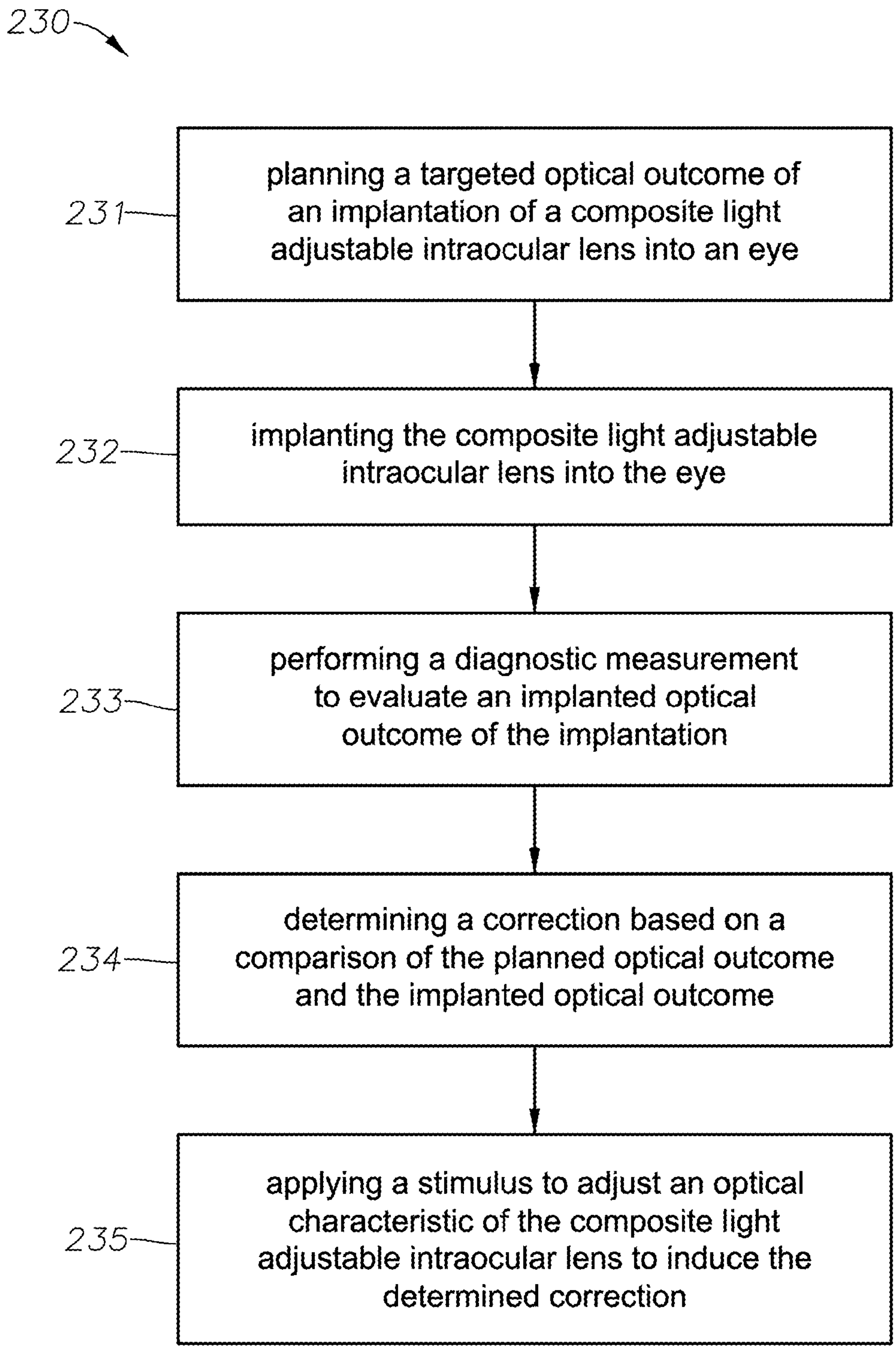
230
231
planning a targeted optical outcome of an implantation of a composite light adjustable intraocular lens into an eye
232
implanting the composite light adjustable intraocular lens into the eye
233
performing a diagnostic measurement to evaluate an implanted optical outcome of the implantation
234
determining a correction based on a comparison of the planned optical outcome and the implanted optical outcome
235
applying a stimulus to adjust an optical characteristic of the composite light adjustable intraocular lens to induce the determined correction

FIG. 9

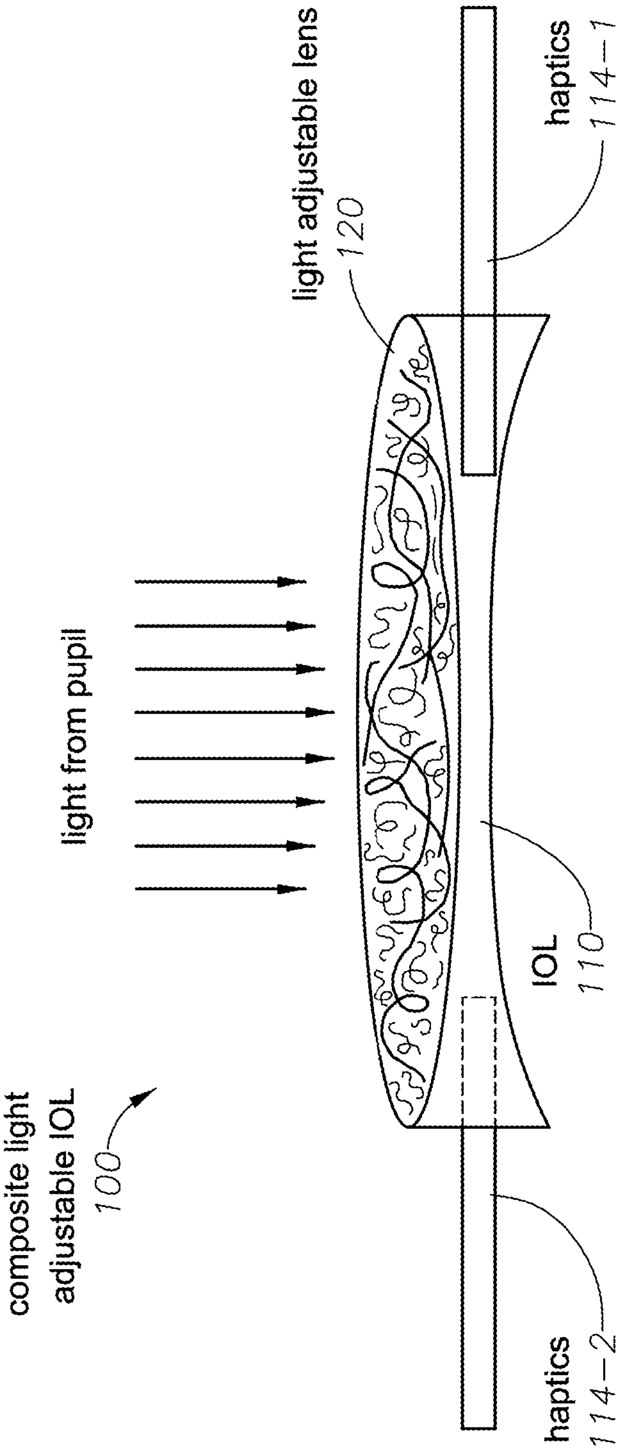
light adjustable lens
120
haptics
114-1
light from pupil
IOL
110
composite light
adjustable IOL
100
haptics
114-2

FIG. 10A

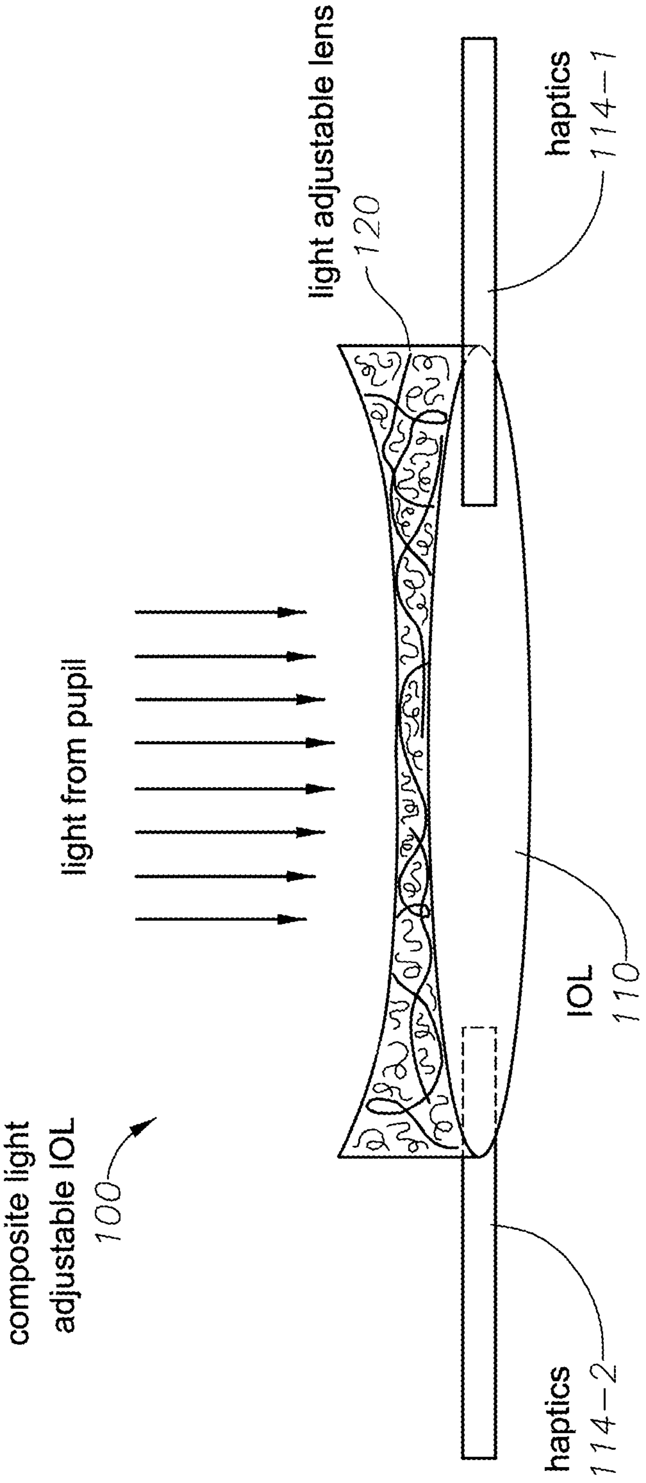
light adjustable lens
120
haptics
114-1
light from pupil
IOL
110
composite light
adjustable IOL
100
haptics
114-2

FIG. 10B

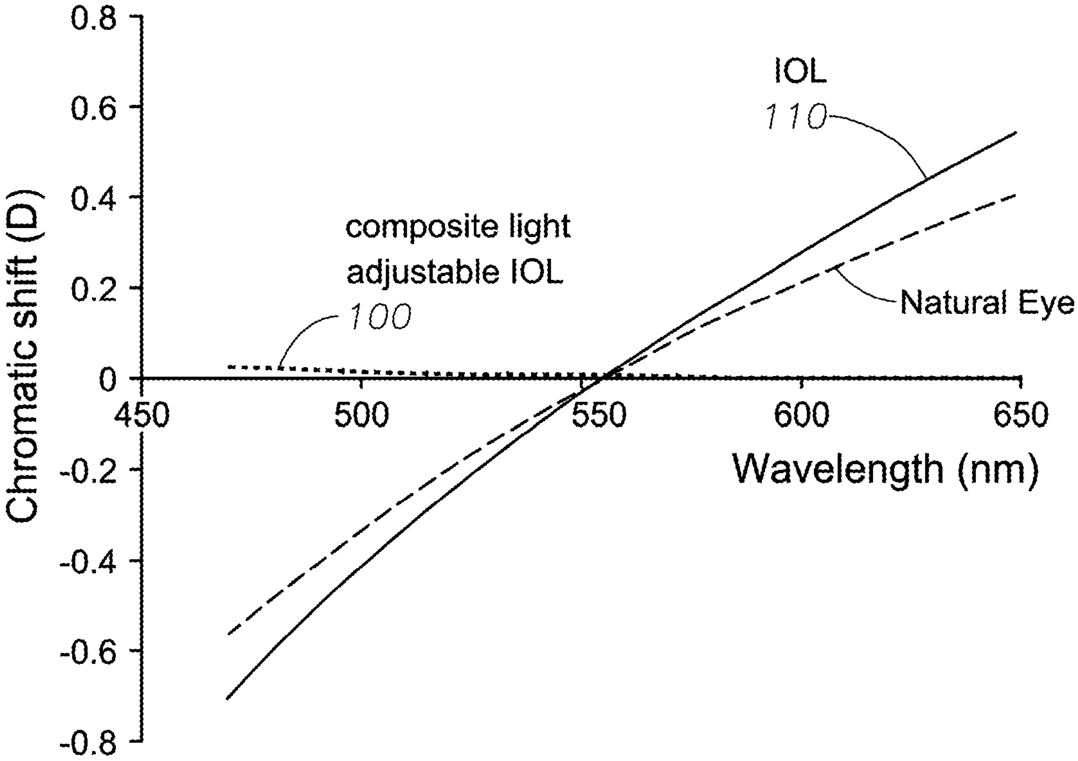
0.8
0.6
0.4
0.2
0
-0.2
-0.4
-0.6
-0.8
Chromatic shift (D)
450
500
550
600
650
Wavelength (nm)
IOL
110
Natural Eye
composite light
adjustable IOL
100

FIG. 11

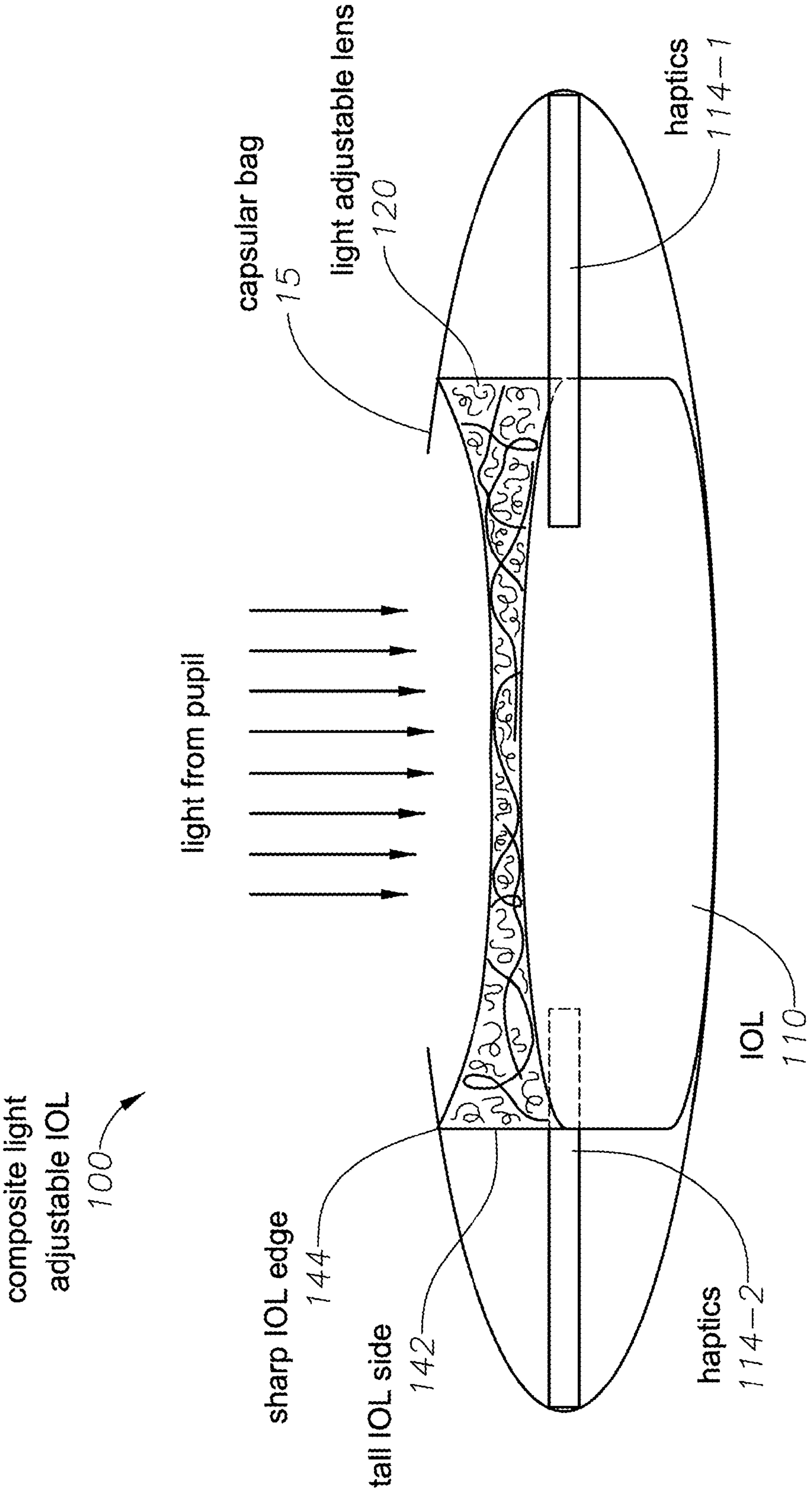
capsular bag
15
light adjustable lens
120
haptics
114-1
light from pupil
composite light
adjustable IOL
100
sharp IOL edge
144
tall IOL side
142
haptics
114-2
IOL
110

FIG. 12A

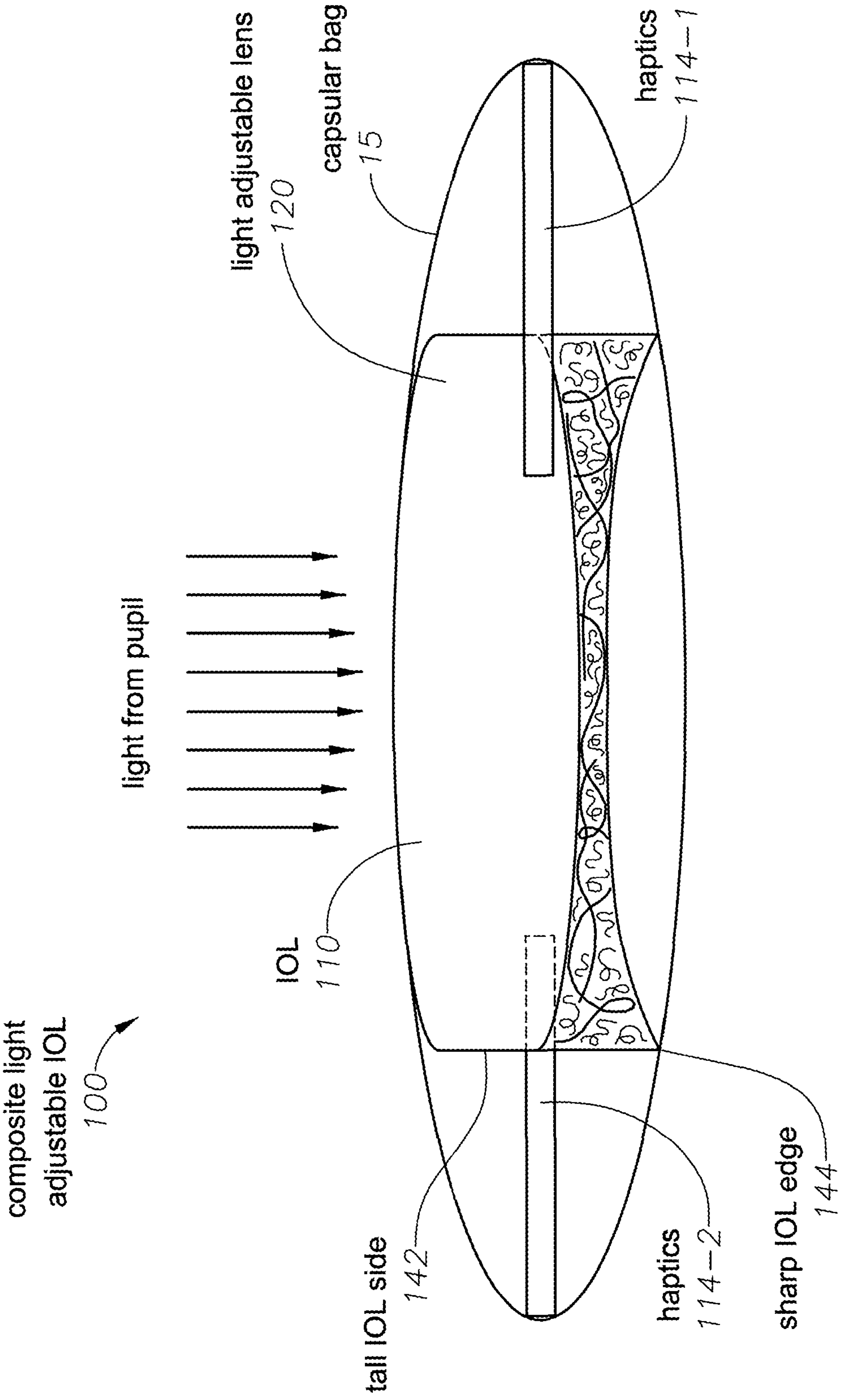
composite light adjustable IOL
100
IOL
110
light from pupil
light adjustable lens
120
capsular bag
15
haptics
114—1
tall IOL side
142
haptics
114—2
sharp IOL edge
144

FIG. 12B

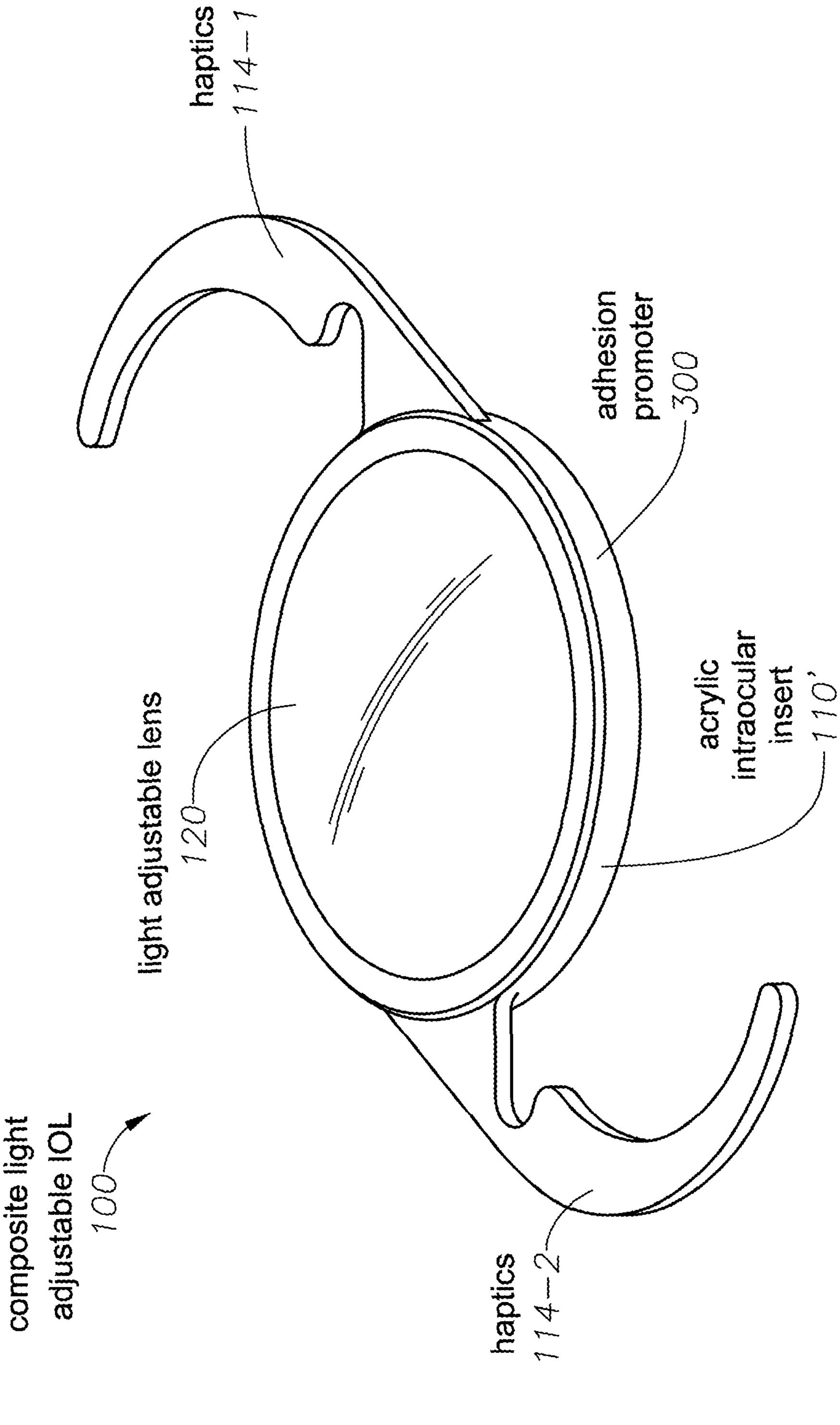
haptics
114-1
adhesion
promoter
300
light adjustable lens
120
acrylic
intraocular
insert
110'
composite light
adjustable IOL
100
haptics
114-2

FIG. 13

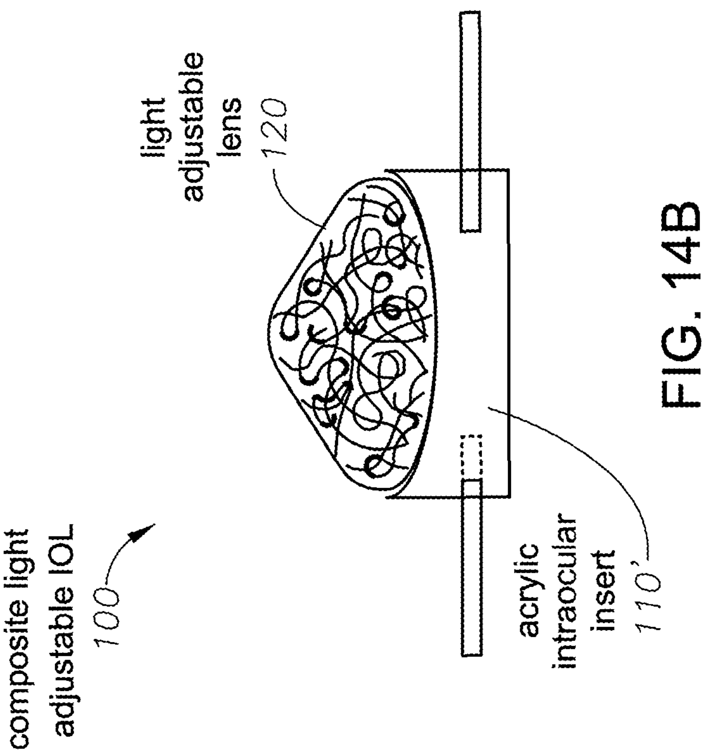


FIG. 14B

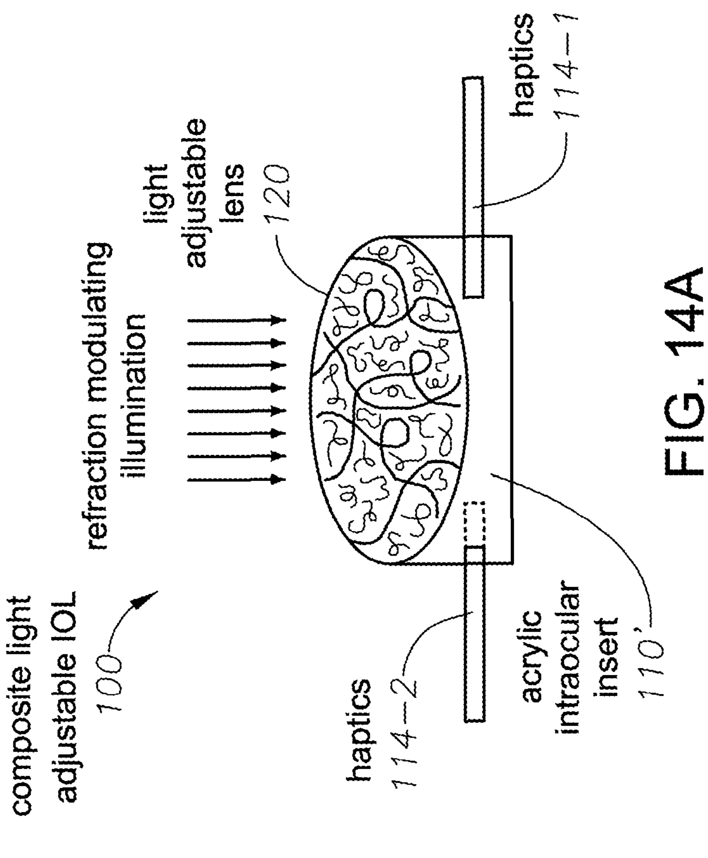


FIG. 14A

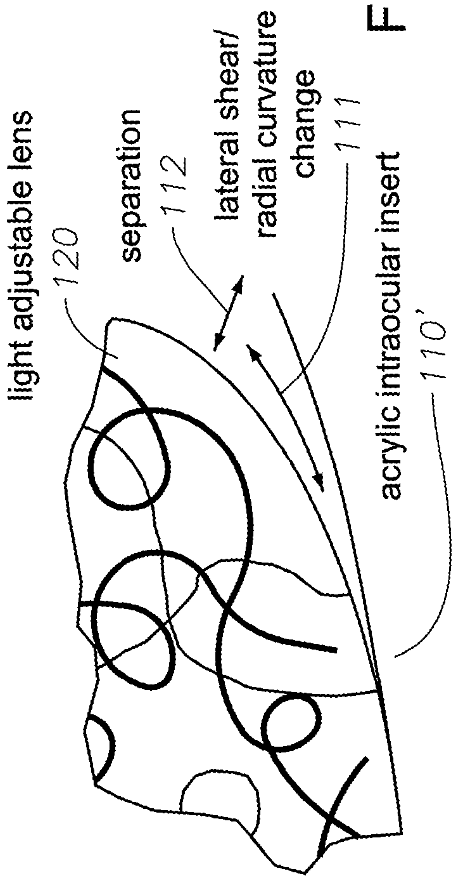


FIG. 14C

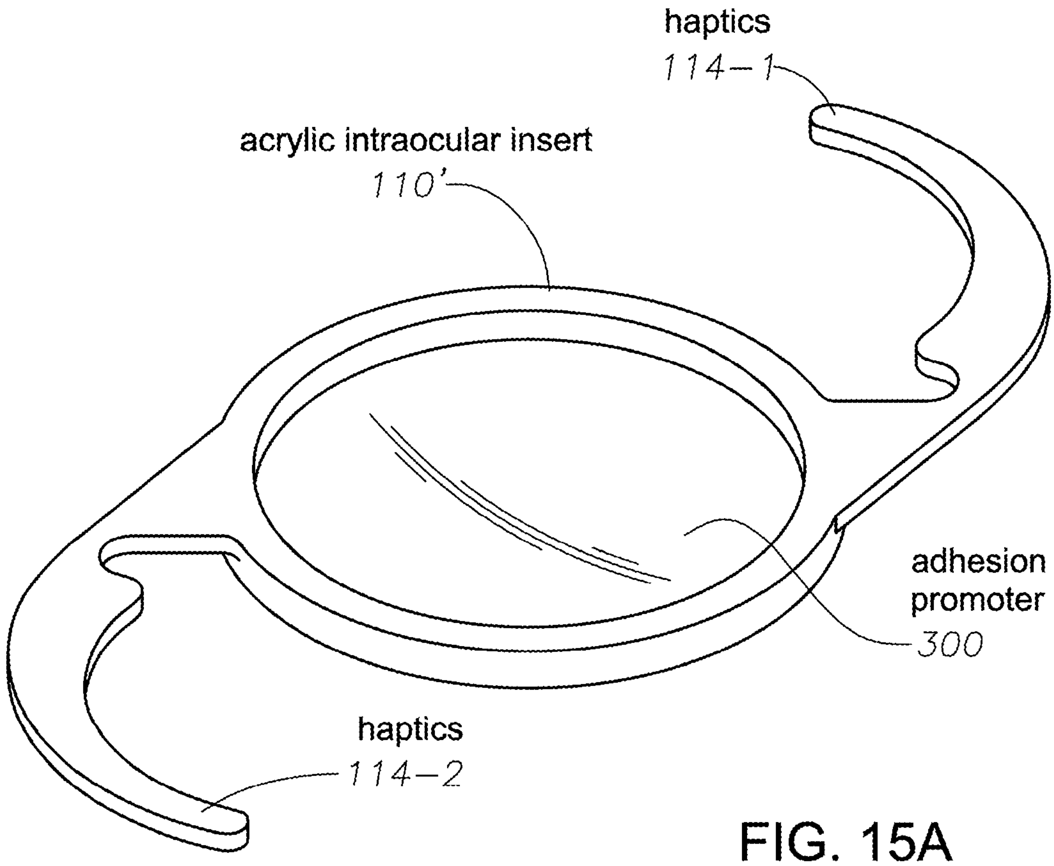


FIG. 15A

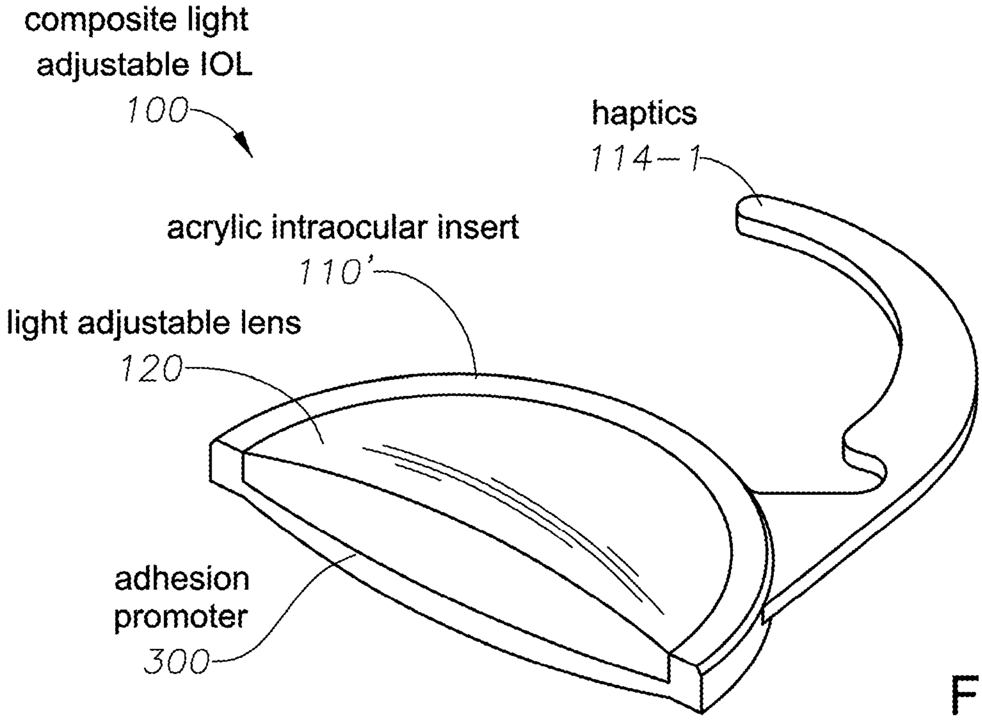


FIG. 15B

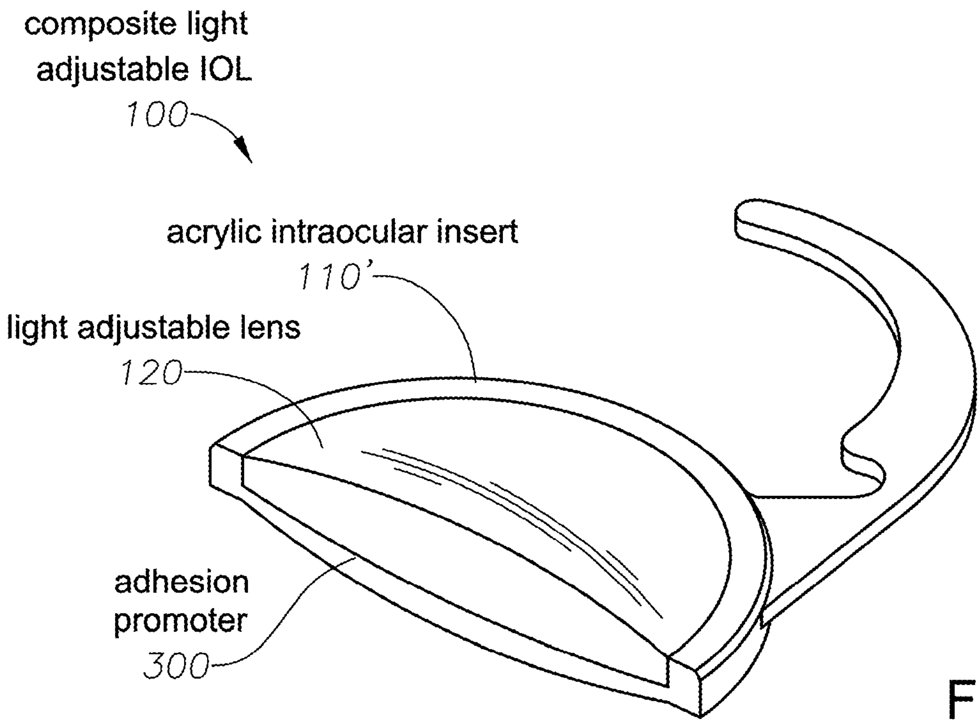


FIG. 16A

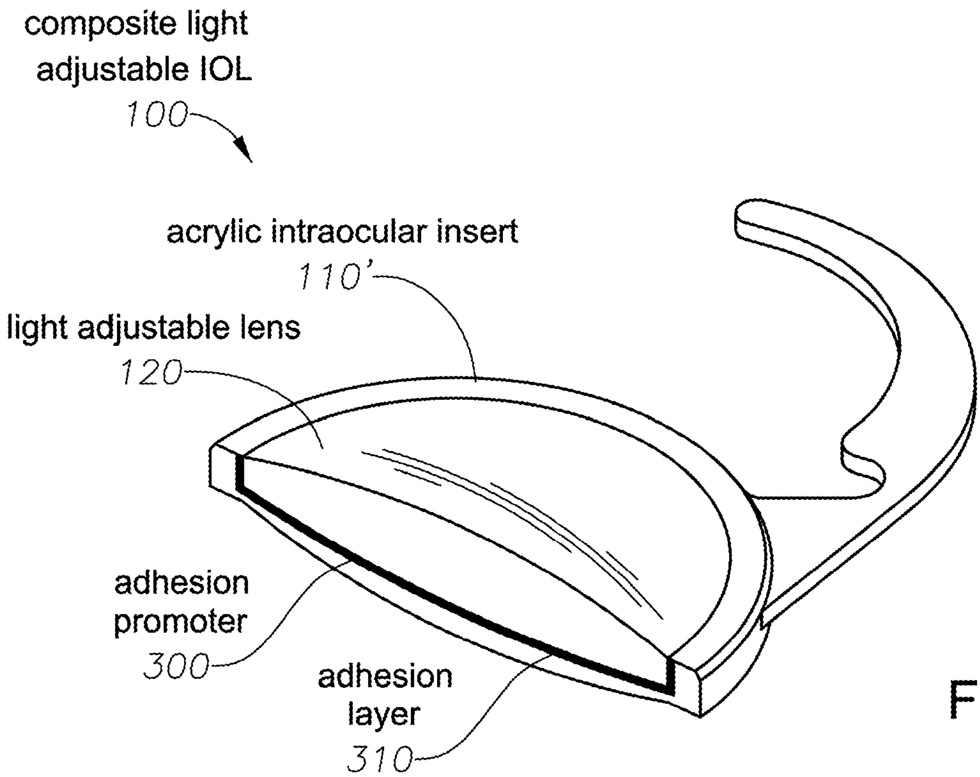


FIG. 16B

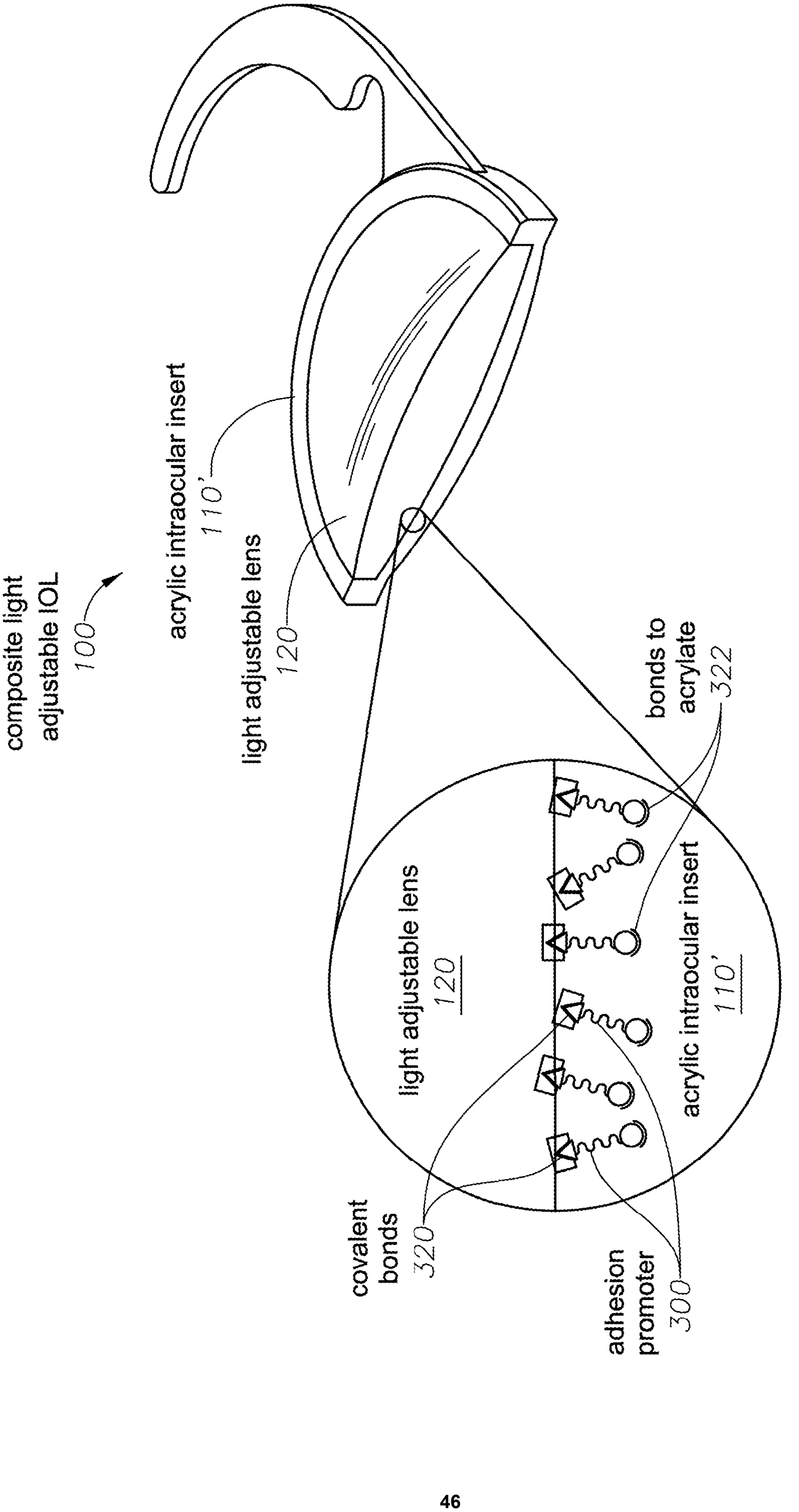
composite light adjustable IOL
100
acrylic intraocular insert
110'
light adjustable lens
120
light adjustable lens
120
acrylic intraocular insert
110'
bonds to acrylate
322
covalent bonds
320
adhesion promoter
300

FIG. 17A

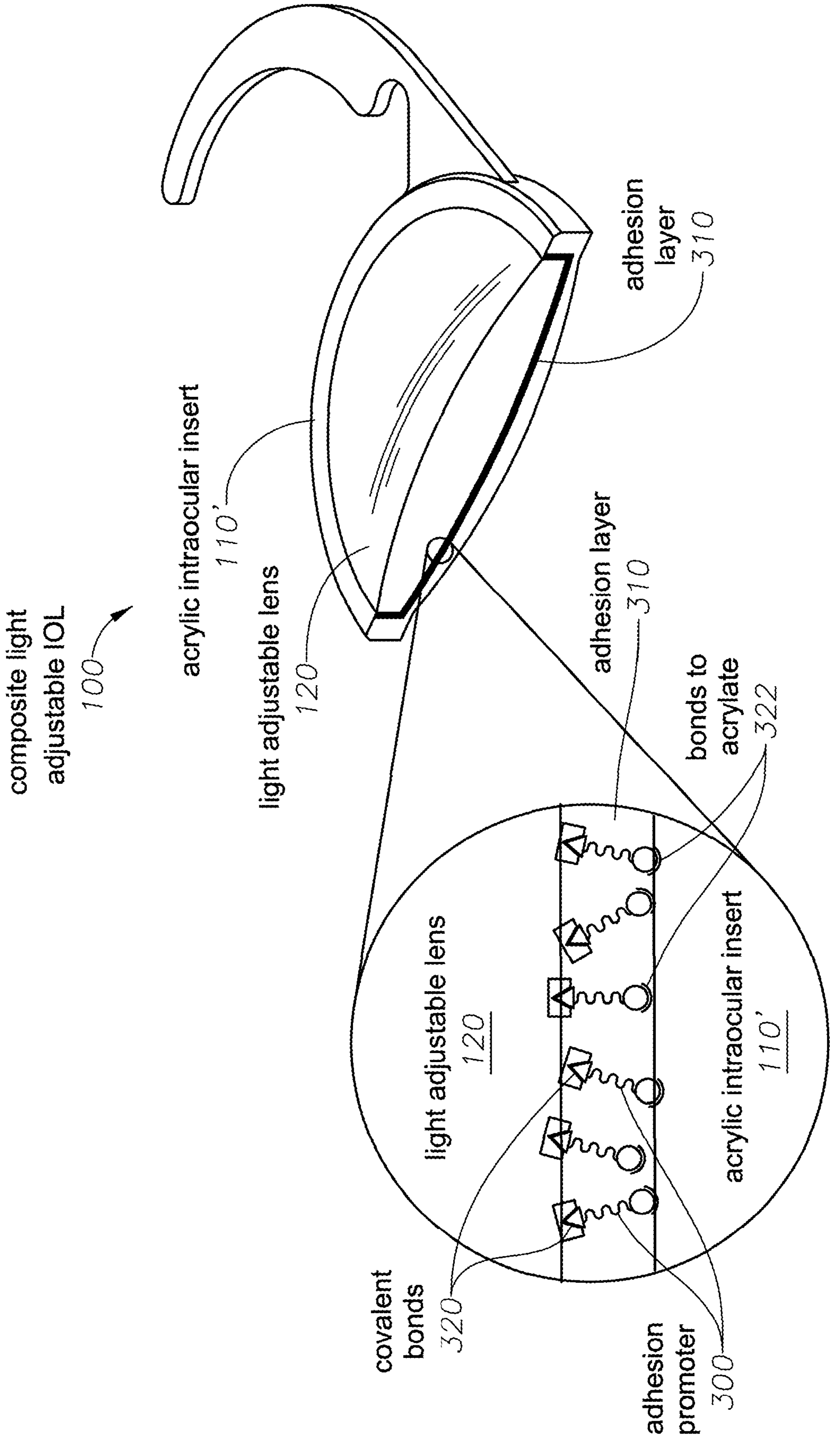
composite light adjustable IOL
100
acrylic intraocular insert
110'
light adjustable lens
120
adhesion layer
310
adhesion layer
310
bonds to acrylate
322
light adjustable lens
120
acrylic intraocular insert
110'
covalent bonds
320
adhesion promoter
300

FIG. 17B

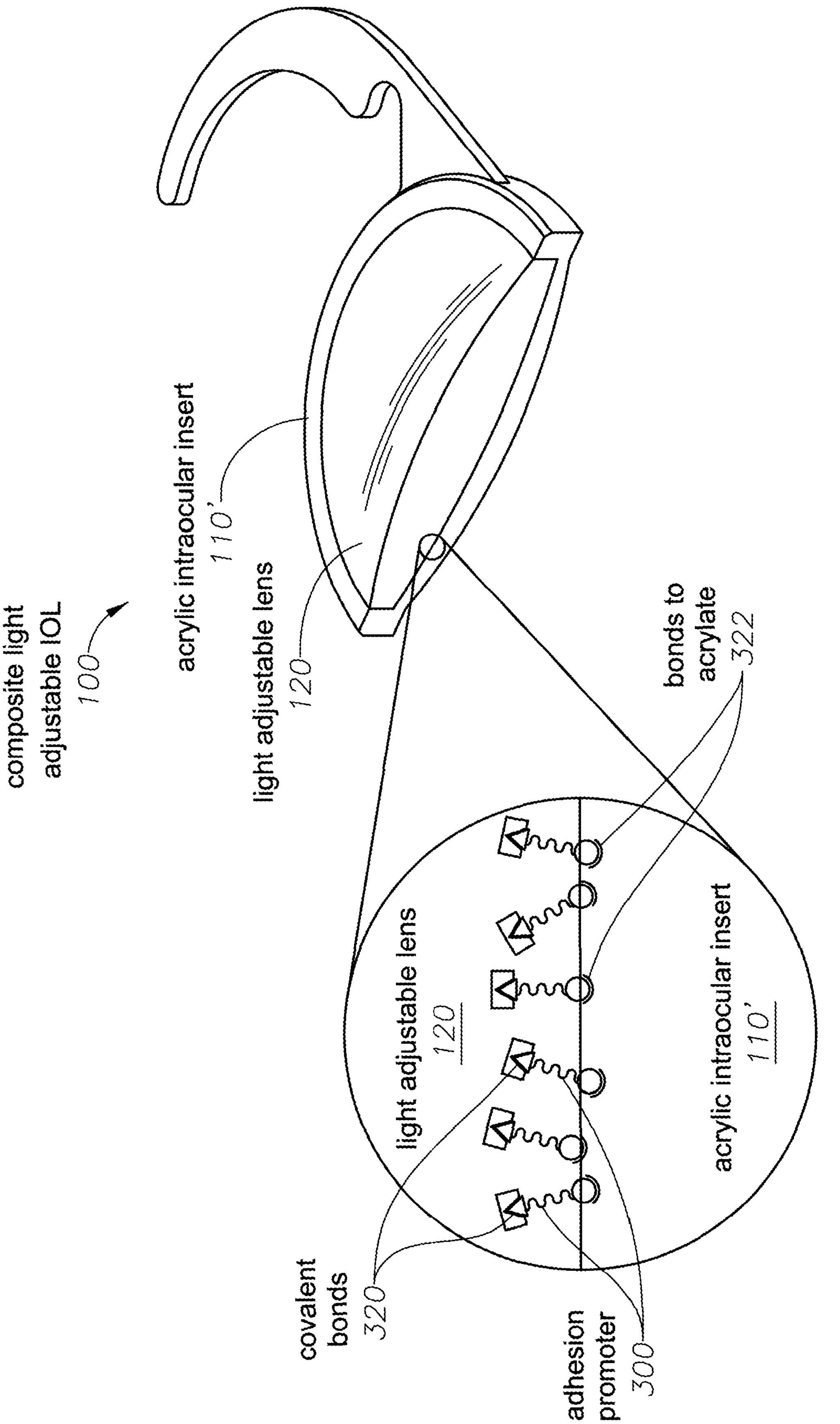
composite light adjustable IOL
100
acrylic intraocular insert
110'
light adjustable lens
120
bonds to acrylate
322
light adjustable lens
120
acrylic intraocular insert
110'
covalent bonds
320
adhesion promoter
300

FIG. 17C

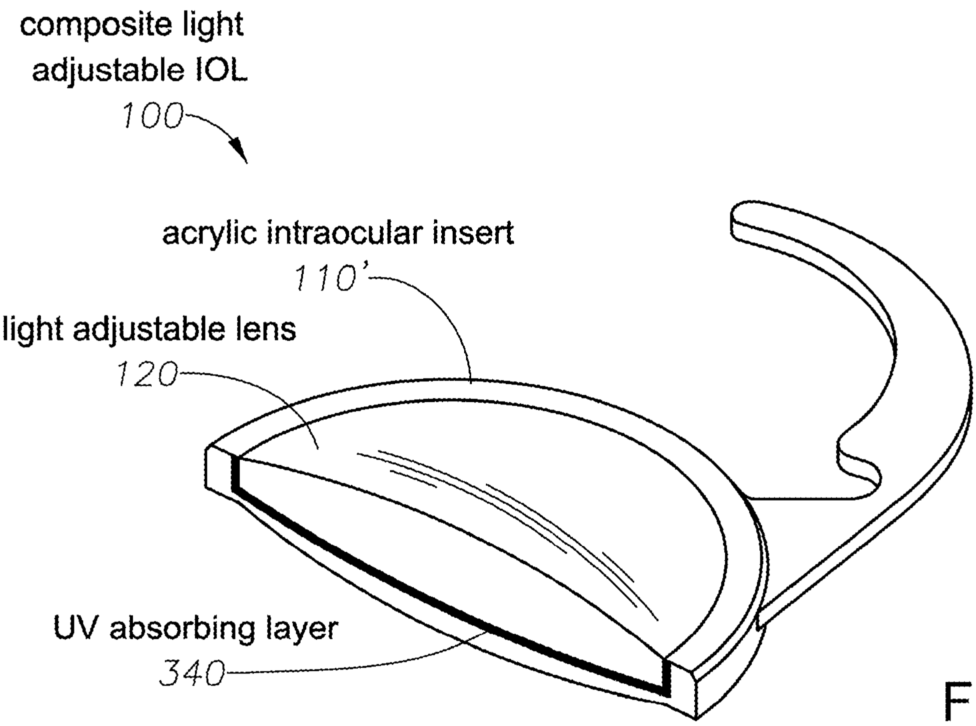


FIG. 18A

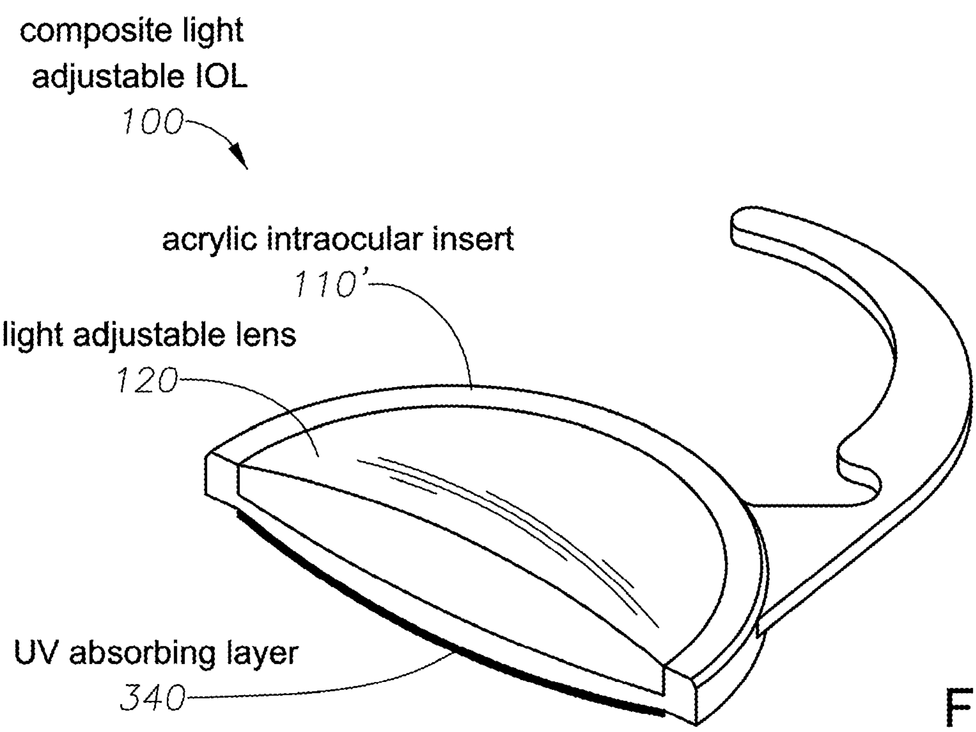


FIG. 18B

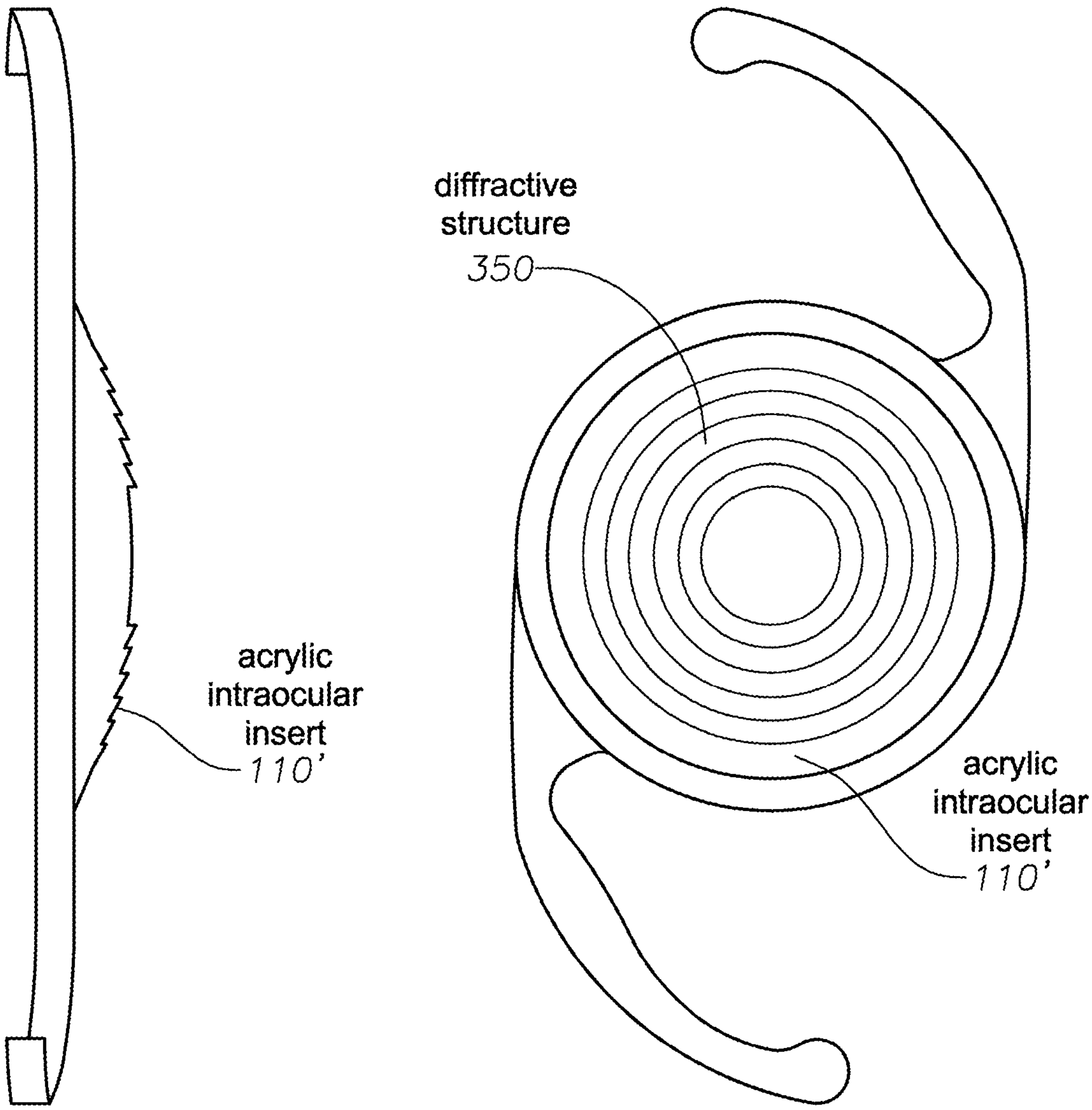
diffractive
structure
350
acrylic
intraocular
insert
110'
acrylic
intraocular
insert
110'

FIG. 19A

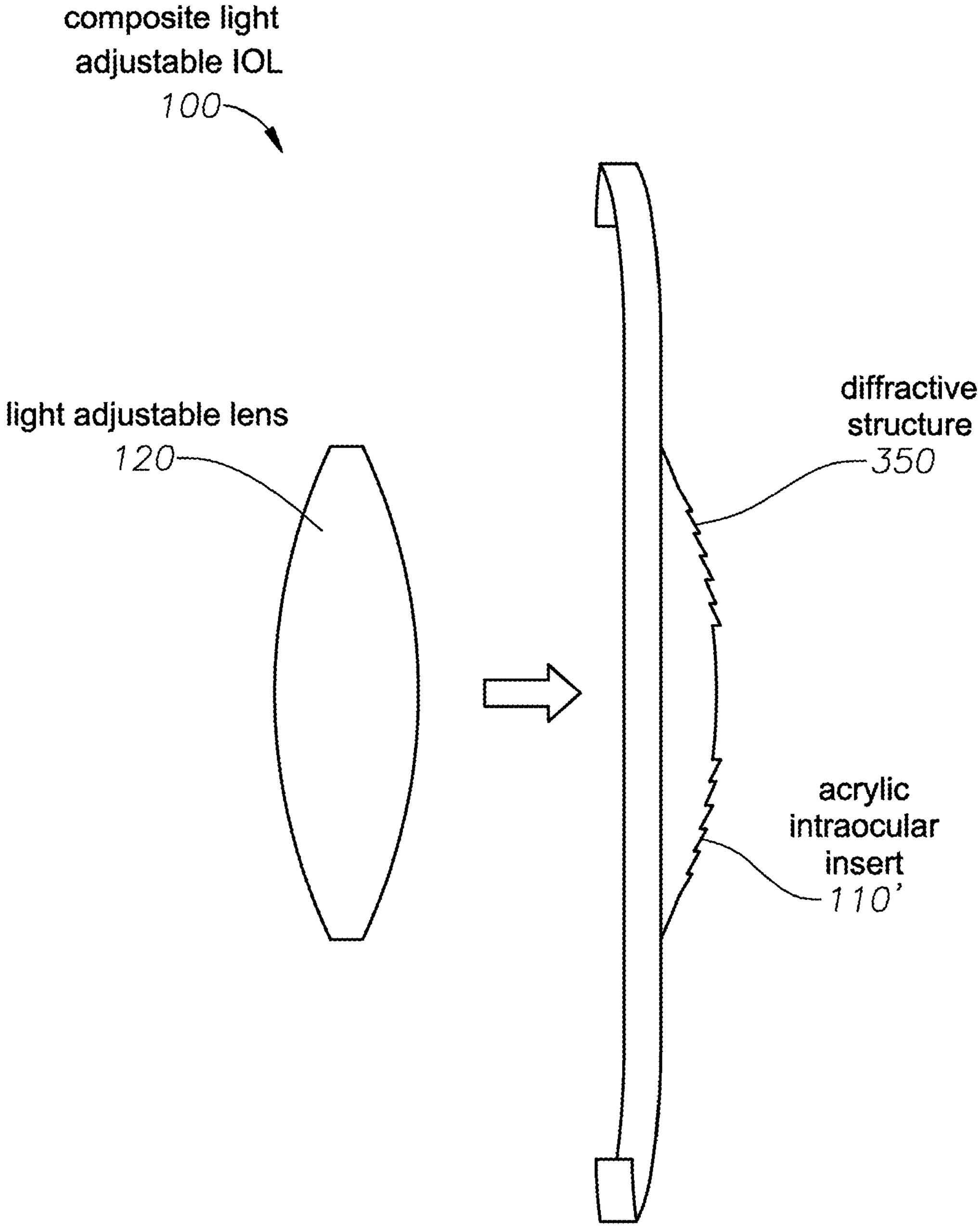
composite light adjustable IOL
100
light adjustable lens
120
diffractive structure
350
acrylic intraocular insert
110'

FIG. 19B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 15607681 B, I. Goldshleger, J. Kondis, R.M. Kurtz, and R. Shrestha
- US 2018338827 A1
- US 6450642 B, J.M. Jethmalani


### Non-patent literature cited in the description


- **E.J. FERNANDEZ** ; **P. ARTAL**. Achromatic doublet intraocular lens for full aberration correction. *Biomedical Optics Express*, 2017, vol. 8, 2396